# FASCICULE DE BREVET EUROPEEN

(11) **EP 3 004 058 B1**
(45) Date de publication et mention de la délivrance du brevet: **10.06.2020**
(21) Numéro de dépôt: 14806879.4
(22) Date de dépôt: 19.05.2014
(51) Int. Cl.: C07D 211/20, A61K 31/445, C07D 211/26, C07D 211/32, A61P 25/28, C07C 211/52, A61K 31/4468

(54) **COMPOSES INHIBITEURS DE L'ACETYLCHOLINESTERASE ET AGONISTES DES RECEPTEURS SEROTONINERGIQUES 5HT4, A EFFET PROMNESIANT, LEURS PROCEDES DE PREPARATION ET COMPOSITIONS PHARMACEUTIQUES LES CONTENANT**
ACETYLCHOLINESTERASEINHIBITORVERBINDUNGEN UND SEROTONERGENE 5HT4-REZEPTORAGONISTEN MIT PROMNESIA-WIRKUNG, VERFAHREN ZUR HERSTELLUNG DAVON UND PHARMAZEUTISCHE ZUSAMMENSETZUNGEN DAMIT
ACETYLCHOLINESTERASE INHIBITOR COMPOUNDS AND 5HT4 SEROTONERGIC RECEPTOR AGONISTS, WITH PROMNESIA EFFECT, METHODS FOR THE PREPARATION THEREOF AND PHARMACEUTICAL COMPOSITIONS CONTAINING SAME

(30) Priorité: 05.06.2013 FR 1355155
(43) Date de publication de la demande: 13.04.2016
(73) Titulaire: Université de Caen, 14000 Caen (FR)
(72) Inventeur: DALLEMAGNE, Patrick, F-14260 Saint Georges d'Aunay (FR); ROCHAIS, Christophe, F-14000 Caen (FR); LECOUTEY, Cédric, F-14000 Caen (FR); BOULOUARD, Michel, F-14440 Douvre la Delivrande (FR); FRERET, Thomas, F-14210 Amaye sur Orne (FR)
(74) Mandataire: Cabinet Netter
(86) Numéro de dépôt international: PCT/FR2014/051149
(87) Numéro de publication internationale: WO 2014/195593

(56) Documents cités:
- EP-A1- 0 774 460
- WO-A1-93/03725
- WO-A1-94/27965
- WO-A1-2007/068739
- WO-A1-2011/099305
- JP-A- H 111 472
- THOMAS FRERET ET AL: "Synergistic effect of acetylcholinesterase inhibition (donepezil) and 5-HTreceptor activation (RS67333) on object recognition in mice", BEHAVIOURAL BRAIN RESEARCH, ELSEVIER, AMSTERDAM, NL, vol. 230, no. 1, 6 février 2012 (2012-02-06), pages 304-308, XP028472750, ISSN: 0166-4328, DOI: 10.1016/J.BBR.2012.02.012 [extrait le 2012-02-14]
- CÉDRIC LECOUTEY ET AL: "Synthesis of dual AChE/5-HT4 receptor multi-target directed ligands", MEDCHEMCOMM, vol. 3, no. 5, 1 janvier 2012 (2012-01-01) , pages 627-634, XP055092302, ISSN: 2040-2503, DOI: 10.1039/c2md20063e
- ITOH K ET AL: "Synthesis and pharmacological evaluation of carboxamide derivatives as selective serotoninergic 5-HT"4 receptor agonists", EUROPEAN JOURNAL OF MEDICINAL CHEMI, EDITIONS SCIENTIFIQUE ELSEVIER, PARIS, FR, vol. 34, no. 4, 1 April 1999 (1999-04-01), pages 329-341, XP004178575, ISSN: 0223-5234, DOI: 10.1016/S0223-5234(99)80083-X
- GASTER L M ET AL: "(1-BUTYL-4-PIPERIDINYL)METHYL 8-AMINO-7-CHLORO-1,4-BENZODIOXANE-5-CARBOX YLATE HYDROCHLORIDE: A HIGHLY POTENT AND SELECTIVE 5-HT4 RECEPTOR ANTAGONIST DERIVED FROM METACLOPRAMIDE", JOURNAL OF MEDICINAL CHEMISTRY, AMERICAN CHEMICAL SOCIETY, vol. 36, 1 January 1993 (1993-01-01), pages 4121-4123, XP000196066, ISSN: 0022-2623, DOI: 10.1021/JM00077A018
- Rong Xu ET AL: "Synthesis, Structure-Affinity Relationships, and Radiolabeling of Selective High-Affinity 5-HT 4 Receptor Ligands as Prospective Imaging Probes for Positron Emission Tomography", Journal of Medicinal Chemistry, vol. 53, no. 19, 14 October 2010 (2010-10-14), pages 7035-7047, XP055092986, ISSN: 0022-2623, DOI: 10.1021/jm100668r
- S Sonda: "Design and synthesis of orally active benzamide derivatives as potent serotonin 4 receptor agonist", Bioorganic & Medicinal Chemistry, vol. 11, no. 19, 15 September 2003 (2003-09-15), pages 4225-4234, XP055092610, ISSN: 0968-0896, DOI: 10.1016/S0968-0896(03)00412-7
- R.M. Eglen ET AL: "Pharmacological characterization of two novel and potent 5-HT4 receptor agonists, RS 67333 and RS 67506, in vitro and in vivo", British Journal of Pharmacology, vol. 115, no. 8, 1 August 1995 (1995-08-01), pages 1387-1392, XP055092326, ISSN: 0007-1188, DOI: 10.1111/j.1476-5381.1995.tb16628.x

## Description

La présente invention concerne de nouveaux dérivés de la 3-(pipéridin-4-yl)-1-(4'-amino-phényl)propan-1-one ainsi que leur utilisation comme médicament à effet promnésiant dans le traitement des maladies neurologiques avec déficits mnésiques.

La présente invention a permis de constater de façon inattendue, que des composés mixtes inhibiteurs catalytiques et périphériques d'acétylcholinestérase et agonistes du récepteur de la sérotonine de type 5-HT4 possèdent des activités promnésiantes, et, en particulier, « anti-Alzheimer ».
De nombreuses pathologies et accidents peuvent provoquer un syndrome amnésique :
- les amnésies progressives associées aux démences telles qu'entre autres la maladie d'Alzheimer (MA) et les démences vasculaires ;
- les traumatismes crâniens (amnésie post-traumatique permanente ou temporaire) ;
- les accidents vasculaires cérébraux ;
- le syndrome de Korsakoff ;
- les tumeurs ou lésions cérébrales ;
- d'autres causes liées à des maladies récurrentes et à long terme telles que l'épilepsie.

La MA est devenue la 4^{ème} cause de décès dans les pays industrialisés et à l'heure actuelle aucun des médicaments sur le marché ne semble en mesure d'exercer un effet curatif. Il s'agit en effet pour la plupart d'entre eux d'inhibiteurs de l'acétylcholinestérase (AChE) dont l'efficacité demeure assez faible et la tolérance médiocre. Il semble nécessaire aujourd'hui d'associer à cet effet symptomatique un effet curatif avec des composés à pluralité d'action (Multi Target Directed Ligands ou MTDL).

Différents MTDL ciblant la MA sont actuellement à l'étude. Cependant aucun parmi ces travaux n'a pour l'instant associé au sein d'une même structure une activité anti-AChE et une activité agoniste 5-HT4, même si des inhibiteurs d'AChE et des agonistes 5-HT4,ⁱ indépendamment les uns des autres ou en associationⁱⁱ ont démontré des propriétés promnésiantes. Or cette dualité d'effet apparaît extrêmement prometteuse dans le cadre d'une chimiothérapie MTDL de la MA. En effet cette approche peut permettre d'exercer avec un seul principe actif un effet symptomatique, lié à la restauration de la transmission cholinergique par l'action inhibitrice catalytique de l'AChE,ⁱⁱⁱ ainsi qu'un double effet potentiellement curatif. Ce dernier serait à mettre au compte d'une part de la promotion du clivage non amyloïdogénique du précurseur de la protéine β-A, et par conséquent de la formation de la protéine sAPPα neurotrophique, en relation avec un effet agoniste 5-HT4.^{iv} Cet effet curatif serait, d'autre part, également imputable à l'inhibition d'un second rôle récemment identifié pour l'AChE, celui de promouvoir l'agrégation amyloïde par interaction entre la protéine β-A et un site périphérique de l'enzyme.

La présente invention concerne le développement de nouveaux médicaments capables d'exprimer, notamment à travers à la fois l'inhibition catalytique et périphérique de l'acétylcholinestérase et un agonisme vis-à-vis des récepteurs à la sérotonine 5-HT4, un véritable effet promnésiant et constitue donc une nouvelle opportunité de traiter entre autres la maladie d'Alzheimer. Il a été découvert que des composés de formule (I) tels que définis ci-après répondent à un tel objectif.

A la connaissance des inventeurs, les composés de formule (I) définis ci-après n'ont à ce jour jamais été décrits et font partie de l'invention à titre de produits nouveaux. Lesdits composés de formule (I) possèdent un mécanisme d'action original mixant à la fois l'inhibition catalytique et périphérique de l'AChE et la stimulation des récepteurs 5-HT4. L'originalité de ce mécanisme d'action pour combattre entre autres la maladie d'Alzheimer fait partie également de l'invention.

Ainsi, selon un premier de ses aspects, la présente invention concerne un composé de formule générale (I) : 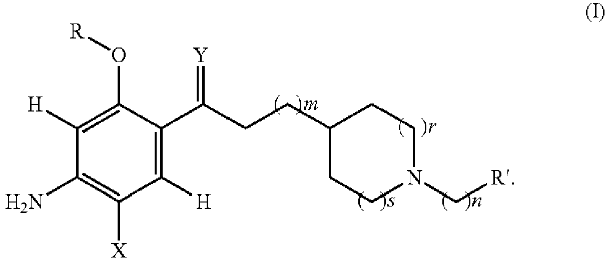 tel que défini dans les revendications.

Comme il ressort des exemples ci-après, des composés de formule (I) selon l'invention présentent des propriétés particulièrement intéressantes à l'égard des symptômes mnésiques, entre autres de la maladie d'Alzheimer.

Selon un mode de réalisation, dans la formule (I), X représente un atome d'halogène.

Selon un autre mode de réalisation, dans la formule (I), Y représente un atome d'oxygène.

Selon au autre mode de réalisation, dans sa formule (I), Z représente un groupe méthylène ou un groupe CH-CH₃.

Selon un autre mode de réalisation, dans sa formule (I), tous les coefficients m, n, r et s ont la valeur 1.

Selon un autre mode de réalisation, dans la formule (I), R représente H, CH₃, CH₂CH₃ ou CH₂-CH₂F.

Selon un autre mode de réalisation, dans la formule (I), R' représente un radical pris dans le groupe formé par les radicaux cyclopropyl, cyclobutyl, cyclopentyl, cyclohexyl, cycloheptyl, et 4-pipéridine.

Selon un autre mode de réalisation, dans la formule (I), R représente un radical méthyl et R' un radical cycloalkyl en C₄-C₇.

L'invention comprend également plusieurs procédés pour préparer les composés de formule (I).

La présente description porte donc aussi sur un premier procédé de préparation des composés de formule (I) pour lesquels R=CH₃, Y=0 et Z, X, m, n, r, s et R' ont les significations précédemment indiquées pour la formule (I), dans lequel
a) on fait réagir du carbonyldiimidazole, puis du manolate d'éthyle de potassium dans le tétrahydrofurane en présence de chlorure de magnésium avec un acide 4-amino-2-méthoxybenzoïque pour obtenir un composé de formule générale (II) 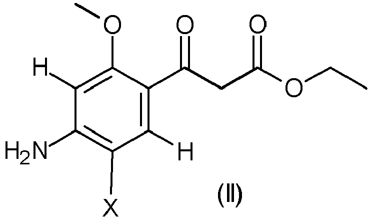 formule dans laquelle X a la même signification que celle indiquée pour la formule (I) ;
b) on fait réagir le composé (II) ainsi obtenu avec un 4-(halogénoalkyl)pipéridine-1-carboxylate de tertiobutyle dans le diméthylformamide en présence de carbonate de potassium, puis avec de l'hydroxyde de potassium dans un mélange éthanol/eau pour obtenir un composé de formule générale (III) 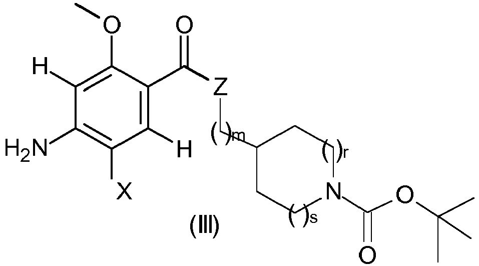 formule dans laquelle Z représente un groupement méthylène et X, m, r et s ont les mêmes significations que celles indiquées pour la formule (I) ;
c) on fait réagir le composé de formule (III) obtenu à l'étape précédente avec de l'acide trifluoracétique dans le dichlorométhane puis avec un halogénure d'alkyle ou de 4-alkylpipéridine N-substituée en présence d'un excès de carbonate de potassium dans le diméthylformamide, ce qui permet d'obtenir les composés de formule (I) désirés.

La description porte aussi sur un deuxième procédé dans lequel
a) on fait réagir un acide 4-amino-3-méthoxybenzoïque avec un 4-(aminoalkyl)pipéridine-1-carboxylate de tetbutyle en présence d'hydroxybenzotriazole, d'éthyl-3-(3-diméthylaminopropyl)carbodiimide et de triéthylamine dans le diméthylformamide, pour obtenir des composés de formule (III) où Z représente un groupement amino secondaire et où X, m, r et s ont les significations indiquées pour la formule (I) ;
b) on fait réagir le composé de formule (III) obtenu dans l'étape a) ci-dessus selon le protocole indiqué à l'étape c) du premier procédé susmentionné, pour obtenir les composés de formule (I) désirés.

La description porte aussi sur un troisième procédé de préparation des composés de formule (I), dans la formule desquels R=CH₃, Y=Z=0 et X, m, n, r, s et R' ont les significations indiquées pour la formule (I), dans lequel on fait réagir un acide 4-amino-3-méthoxybenzoïque avec une 1-alkyl-4(hydroxyalky)pipéridine en présence de carbonylediimidazole et de carbonate de potassium dans le tétrahydrofurane pour obtenir le composé de formule (I) désiré.

L'invention porte aussi sur le composé 2-chloro-4-[[2-[1-(cyclohexylméthyl)-4-pipéridyl]éthyl]-N-méthoxycarbonimidoyl]-5-méthoxyaniline.

L'invention porte aussi sur un quatrième procédé de préparation des composés dans la formule (I) desquels R=H et X, Y, m, n, r, s, et R' ont les significations indiquées pour la formule (I), caractérisé en ce que l'on fait réagir un composé de formule (I) dans la formule duquel R représente un groupe méthyl avec du chlorure d'aluminium en présence d'iodure de sodium dans l'acétonitrile, pour obtenir le composé de formule (I) désiré.

L'invention porte aussi sur un cinquième procédé de préparation des composés de formule (I) dans la formule desquels R représente un radical alkyl en C1-C3, en chaine droite ou ramifiée, pouvant porter un ou plusieurs atomes de fluor, X, Y, m, n, r, s, et R' ayant les significations indiquées pour la formule (I), caractérisé en ce que l'on fait réagir un composé de formule (I), dans lequel R = H et X, Y, m, n, r, s, et R' ayant les significations précédemment indiquées pour la formule (I), avec un halogénure ou un tosylate d'alkyl en C1-C3, en chaine droite ou ramifiée, pouvant porter un ou plusieurs atomes de fluor, en présence de carbonate de potassium, dans un solvant approprié.

L'invention porte aussi sur un sixième procédé de préparation des composés de formule (I) telle que décrit ci-dessus dans lesquels Y est un groupe oxime éventuellement O-substitué, X, m, n et R' étant tels qu'indiqués pour la formule (I), caractérisé en ce que l'on fait réagir un composé dans la formule (I) duquel X, m, n et R' ont les significations indiquées ci-dessus et Y = O, avec un sel d'hydroxylamine O-substituée en présence de carbonate de calcium dans un mélange éthanol/eau, pour obtenir le composé de formule (I) désiré.

La description porte aussi sur un composé de formule (II) obtenu à la fin de l'étape a) du premier procédé de préparation ci-dessus mentionné et sur un composé de formule (III) obtenu à la fin de l'étape b) de ce même procédé de préparation.

L'invention porte enfin sur une composition pharmaceutique telle que définie dans les revendications.

Selon un premier aspect de l'invention, la composition, qui vient d'être définie, comporte un excipient pharmaceutiquement acceptable.

Selon un autre aspect de l'invention, le(s) composé(s) de formule (I), que contient la composition, est (sont) un (des) sel(s) pharmaceutiquement acceptable(s).

Selon un autre aspect de l'invention, la composition contient au moins un principe actif ayant une action inhibitrice d'acétylcholinestérase et choisi dans le groupe formé par :
a) la 1,2,3,4-Tétrahydroacridin-9-amine
b) le (RS)-2-[(1-benzyl-4-pipéridyl)méthyl-5,6-diméthoxy-2,3-dihydroindén-1-one
c) la (S)-N-éthyl-N-méthyl-3-[(1-diméthylamino)éthyl]-phényl carbamate et
d) la 4aS,6R,8aS)-5,6,9,10,11,12-hexahydro-3-méthoxy-11-méthyl-4aH-[1]benzofuro[3a,3,2-ef][2]benzazépin-6-ol

Selon un autre aspect de l'invention, la composition contient au moins un agoniste partiel des récepteurs 5-HT4 choisi dans le groupe formé par :
a) le 1-(4-amino-5-chloro-2-méthoxyphényl)-2-[1-butyl-4-piperidyl]propan-1one,
b) le N-(2-(4-(3-(4-amino-5-chloro-3-méthoxyphényl)-3-oxypropyl)pipéridin-1-yl)éthyl)méthane sulfonamide,
ledit agoniste étant dans une proportion comprise entre 10/90 et 90/10 par rapport au(x) principe(s) actif(s) constitué(s) par au moins un composé tel que défini dans les revendications.

Selon un autre aspect de l'invention, la composition pharmaceutique est destinée à être administrée à des mammifères et a un effet promnésiant utilisable dans les maladies neurologiques avec déficit amnésique.

Selon un autre aspect de l'invention, la composition est utilisable dans le traitement de la maladie d'Alzheimer chez un sujet humain.

La composition peut être utilisée à des doses journalières d'actif(s) de formule (I) comprises entre 1 et 20 mg/kg de poids corporel du mammifère à traiter, de préférence de 2mg/kg.

La composition peut être administrée à des doses de 10 à 1000 mg par jour, les composés de formule (I) étant administrés de préférence à raison de 0,2 à 2mg une à cinq fois par jour.

La composition peut être administrée par voie orale, notamment sous forme de comprimés, gélules, microcapsules, poudres, granulés, sirops, solutions ou suspensions prises en buccal ou sublingual ou par voie sous-cutanée, intramusculaire ou intraveineuse.

Selon un autre aspect de l'invention, le principe actif est conditionné sous forme de mélange avec des agents de dispersion, des agents mouillants, des agents de mise en suspension, des édulcorants ou des correcteurs de goût.

Selon un autre aspect de l'invention, la composition est administrée par voie parentérale, sous forme de suspension aqueuse, de solution saline, de solution stérile ou de solution injectable.

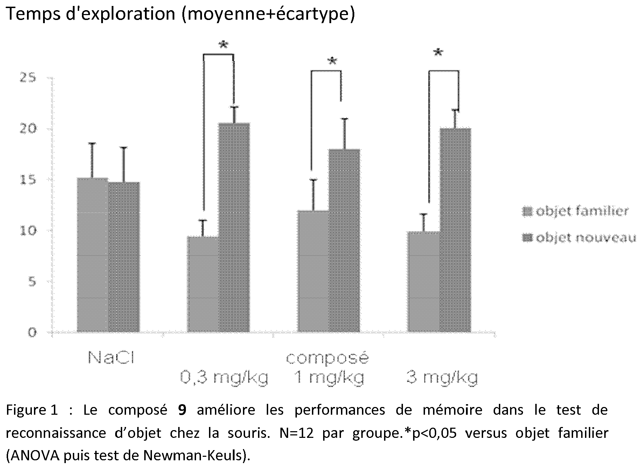

Pour mieux faire comprendre l'objet de l'invention et illustrer celle-ci de façon non limitative, on va décrire ci-après un certain nombre d'exemples. Pour faciliter le repérage des différents composés illustrés dans les exemples, on a fourni, ci-après, un tableau 1 correspondant aux composés de formule (I), un tableau 2 correspondant aux composés de formule (II) et un tableau 3 correspondant aux composés de formule (III) et en donnant, dans chacun de ces tableaux, les significations des différents substituants pour chacun des exemples fournis dans le texte ci-après. Les numéros apparaissant dans ces exemples, correspondent aux composés portant respectivement les mêmes numéros dans les tableaux ci-dessus. Les chiffres apparaissant dans le texte en exposant, correspondent à des références utilisées pour la réalisation des exemples. La figure 1 fournit les résultats obtenus sur les souris pour l'amélioration des performances de mémoire.

**Tableau 1 : composés de formule (I) ci-dessous**

| | | | | | | | | | |
|---|---|---|---|---|---|---|---|---|---|
| 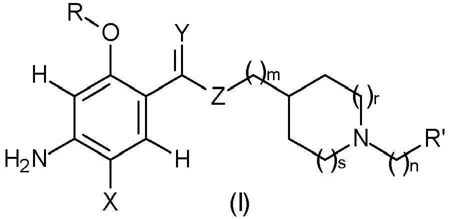 | | | | | | | | | |

| Composé | X | Y | Z | m | n | r | s | R | R' |
|---|---|---|---|---|---|---|---|---|---|
| **1*** | H | O | CH₂ | 1 | 1 | 1 | 1 | Me | n-propyl |
| **2*** | Br | O | CH₂ | 1 | 1 | 1 | 1 | Me | n-propyl |
| **3*** | I | O | CH₂ | 1 | 1 | 1 | 1 | Me | n-propyl |
| **4** | H | O | CH₂ | 1 | 1 | 1 | 1 | Me | cyclohexyl |
| **5** | F | O | CH₂ | 1 | 1 | 1 | 1 | Me | cyclohexyl |
| **6** | Cl | O | CH₂ | 1 | 1 | 1 | 1 | Me | cyclopropyl |
| **7** | Cl | O | CH₂ | 1 | 1 | 1 | 1 | Me | cyclobutyl |
| **8** | Cl | O | CH₂ | 1 | 1 | 1 | 1 | Me | cyclopentyl |
| **9** | Cl | O | CH₂ | 1 | 1 | 1 | 1 | Me | cyclohexyl |
| **10*** | Cl | O | CHCH | 1 | 1 | 1 | 1 | Me | cyclohexyl |
| **11** | Cl | O | CH₂ | 1 | 1 | 1 | 1 | Me | 2-methylcyclohexyl |
| **12** | Cl | O | CH₂ | 1 | 1 | 1 | 1 | Me | cycloheptyl |
| **13** | Cl | O | CH₂ | 1 | 1 | 1 | 1 | Me | 4-pipéridine |
| **14** | Br | O | CH₂ | 1 | 1 | 1 | 1 | Me | cyclohexyl |
| **15** | I | O | CH₂ | 1 | 1 | 1 | 1 | Me | cyclohexyl |
| **16** | Cl | O | CH₂ | 1 | 2 | 1 | 1 | Me | cyclohexyl |
| **17*** | Cl | O | NH | 1 | 1 | 1 | 1 | Me | cyclohexyl |
| **18*** | Br | O | NH | 1 | 1 | 1 | 1 | Me | cyclohexyl |
| **19*** | I | O | NH | 1 | 1 | 1 | 1 | Me | cyclohexyl |
| **20*** | Cl | O | O | 1 | 1 | 1 | 1 | Me | cyclohexyl |
| **21** | Cl | O | CH₂ | 1 | 1 | 1 | 1 | H | cyclohexyl |
| **22** | Cl | O | CH₂ | 1 | 1 | 1 | 1 | Et | cyclohexyl |
| **23** | Cl | O | CH₂ | 1 | 1 | 1 | 1 | F(CH₂)₂ | cyclohexyl |
| **24** | Cl | N-OMe | CH₂ | 1 | 1 | 1 | 1 | Me | cyclohexyl |

| | | | | | | | | | |
|---|---|---|---|---|---|---|---|---|---|
| *** : Non-couvert par l'invention** | | | | | | | | | |

**Tableau 2 : composés de formule (II)**

| | |
|---|---|
| 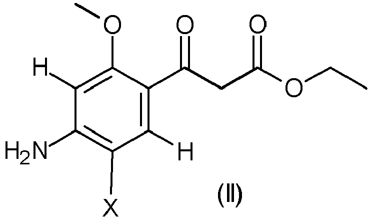 | |

| Composé n° | X |
|---|---|
| **25** | H |
| **26** | F |
| **27** | Cl |
| **28** | Br |
| **29** | I |

**Tableau 3 : composés de formule (III)**

| | | | | | |
|---|---|---|---|---|---|
| 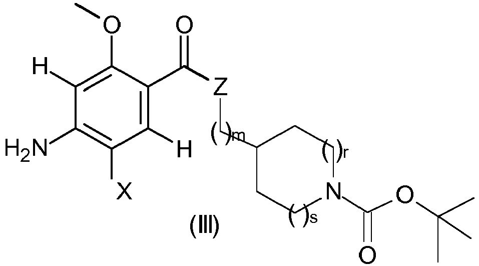 | | | | | |

| Composé n° | X | Z | m | r | s |
|---|---|---|---|---|---|
| **30** | H | CH₂ | 1 | 1 | 1 |
| **31** | Cl | CHCH₃ | 1 | 1 | 1 |
| **32** | F | CH₂ | 1 | 1 | 1 |
| **33** | Br | CH₂ | 1 | 1 | 1 |
| **34** | I | CH₂ | 1 | 1 | 1 |
| **35** | Br | NH | 1 | 1 | 1 |
| **36** | I | NH | 1 | 1 | 1 |

### Références pour la réalisation des exemples :

¹ Eustache, F., Desgranges, B. Concepts et modèles en neuropsychologie de la mémoire. (2003). In Meulemans, T., Desgranges, B., Adam, S., Eustache, F. (eds.). Evaluation et prise en charge des troubles mnésiques. Marseille : Solal.
¹ Mingaud F., Mormede C., Etchamendy N., Mons N., Niedergang B., Wietrzych M., Pallet V., Jaffard R., Krezel W., Higueret P., Marighetto A., 2008 J. Neurosci. 28, 279-291.
¹ Levallet G, Hotte M, Boulouard M, Dauphin F (2009) Psychopharmacology (Berl). 202:125-39.
¹ Moser, P.C., Bergis, O.E., Jegham, S., Lochead, A., Duconseille, E., Terranova, J.P. J Pharmaco Exp Ther 2002 ; 302 :731-741.
¹ Birks, J. Cochrane Database Syst. Rev. 2006, CD005593.
¹ Lezoualc'h, F. Exp. Neurol. 2007, 205, 325-329.
¹ Holzgrabe, U.; Kapkova, P.; Alptuzun, V.; Scheiber, J.; Kugelmann, E. Expert Opin. Ther. Targets 2007, 11, 161-179.
¹ Clark, R. D.; Eglen, R.; Jahangir, A.; Miller, A. B. ; Gardner, J. O. PCT Int. Appl. 1994, WO9427965
¹ Sonda, S. ; Kawahara, T. ; Murozono, T. ; Sato, N. ; Asano, K.; Haga, K. Bioorg. Med. Chem. 2003, 11(19), 4225-4234
¹ Yang, W. ; Ruan, Z. ; Wang, Y. ; Van Kirk, K.; Ma, Z.; Arey, B. J.; Cooper, C. B.; Seethala, R.; Feyen, J. H. M.; Dickson Jr, J. K. J. Med. Chem. 2009, 52 (4), 1204 -1208
¹ Kaspi, J. ; Lerman, O. ; Arad, O. ; Alnabari, M.; Sery, Y. Eur. Pat. Appl. 2004, 1386607

### Exemple 1 (Non-couvert par l'invention)

### 1-(4-amino-2-méthoxyphényl)-3-[1-butyl-4-pipéridyl]propan-1-one

A 184 mg de 4-[3-(4-amino-2-méthoxy)-3-oxopropyl]pipéridine-1-carboxylate de *tert*-butyle (0,51 mmol) en solution dans 1 ml de DCM est ajouté 1 ml de TFA. Le milieu réactionnel est agité à TA pendant 15 minutes puis concentré à l'évaporateur rotatif sous pression réduite. Le résidu est repris dans NaHCO₃ saturée puis extrait 3 fois à l'AcOEt. Les phases organiques sont regroupées puis lavées à la saumure. La phase organique est ensuite séchée et concentré pour obtenir 42 mg de produit déprotégé. Celui-ci est directement engagé dans la réaction d'alkylation en le solubilisant dans 3 ml de DMF auquel on ajoute 28 mg de K₂CO₃ (0,21 mmol) et 20 µL d'iodobutane (0,18 mmol). Le nouveau milieu réactionnel est placé à 110°C pendant 2h. Après dilution dans l'AcOEt, le mélange est lavé 4 fois à la saumure, séché sur MgSO₄ puis concentré. Le résidu obtenu est purifié sur colonne de gel de silice (DCM/AcOEt 10/0 à 0/10 puis MeOH 5%). Le produit obtenu est obtenu avec un rendement de 22%.
C₁₉H₃₀N₂O₂
PF = 80°C
RMN ¹H (CDCl₃) : 0,89 (t, J = 7,3Hz, 3H, CH₃), 1,27 (m, 5H, 3 CH, CH_{2 BUT}), 1,45 (m, 2H, CH_{2 BUT}), 1,56 (m, 2H), 1,68 (m, 2H), 1,85 (m, 2H, 2 CHN), 2,27 (m, 2H, CH₂N), 2,90 (m, 4H, CH₂, 2 CHN), 3,83 (s, 3H, CH₃), 4,03 (s, 2H, NH₂), 6,13 (d, J = 2,0Hz, 1H, H_{Ar}), 6,24 (dd, J = 8,7Hz, J = 2,0Hz, 1H, H_{Ar}), 7,69 (d, J = 8,7Hz, 1H, H_{Ar},).
IR (KBr, cm⁻¹) : 3438,2, 3255,4, 3228,4, 2952,1, 2926,8, 2932,8, 2856,9, 1726,5, 1640,7, 1594,1, 1468,1, 1434,3, 1272,4, 1259,8, 1212,6, 1177,1, 1030,2.

### Exemple 2 (Non couvert par l'invention)

### 1-(4-amino-5-bromo-2-méthoxyphényl)-3-[1-butyl-4-pipéridyl] propan-1-one

Le composé 3 est préparé suivant le mode opératoire de l'exemple 1 décrit ci-dessus en considérant les quantités suivantes :
4-[3-(4-amino-5-bromo-2-méthoxy)-3-oxopropyl]pipéridine-1-carboxylate de tertbutyle: 90 mg (0,19 mmol)
Acide trifluoroacétique : 1 ml
DCM : 1 ml
iodobutane : 26 µL (0,23 mmol)
K₂CO₃ : 34 mg (0,25 mmol)
DMF : 4 ml
Le composé attendu est obtenu avec un rendement de 50% et se présente sous la forme d'une poudre jaune pâle.
Il possède les caractéristiques suivantes :
C₁₉H₂₉BrN₂O₂
PF = 205°C
RMN ¹H (CDCl₃) : 0,91 (t, J = 7,3Hz, 3H, CH₃), 1,29 (m, 5H, 3 CH, CH_{2 BUT}), 1,48 (m, 2H, CH_{2 BUT}), 1,58 (m, 2H), 1,70 (m, 2H), 1,89 (m, 2H, 2 CHN), 2,31 (m, 2H, CH₂N), 2,91 (m, 4H, CH₂, 2 CHN), 3,84 (s, 3H, CH₃), 4,55 (s, 2H, NH₂), 6,27 (s, 1H, H_{Ar}), 7,93 (s, 1H, H_{Ar}).
IR (KBr, cm⁻¹) : 3436,2, 3255,8, 3156,4, 2961,4, 2932,8, 2873,5, 2633,0, 2516,0, 1725,7, 1655,3, 1619,3, 1584,6, 1461,4, 1417,0, 1300,1, 1253,4, 1216,2, 1179,2, 1044,2.

### Exemple 3 (Non-couvert par l'invention)

### 1-(4-amino-5-iodo-2-méthoxyphényl)-3-[1-butyl-4-pipéridyl] propan-1-one

Le composé 4 est préparé suivant le mode opératoire de l'exemple 1 décrit ci-dessus en considérant les quantités suivantes :
4-[3-(4-amino-5-iodo-2-méthoxy)-3-oxopropyl]pipéridine-1-carboxylate de tertbutyle: 124 mg (0,25 mmol)
Acide trifluoroacétique : 1 ml
DCM : 1 ml
iodobutane : 28 µL (0,25 mmol)
K₂CO₃ : 37 mg (0,27 mmol)
DMF : 4 ml
Le composé attendu est obtenu avec un rendement de 45% et se présente sous la forme d'une poudre jaune pâle.
Il possède les caractéristiques suivantes :
C₁₉H₂₉IN₂O₂
PF = 130°C
RMN ¹H (CDCl₃) : 0,91 (t, J = 7,8Hz, 3H, CH₃), 1,27 (m, 5H, 3 CH, CH_{2 BUT}), 1,47 (m, 2H, CH_{2 BUT}), 1,59 (m, 2H), 1,70 (m, 2H), 1,87 (m, 2H, 2 CHN), 2,29 (m, 2H, CH₂N), 2,91 (m, 4H, CH₂, 2 CHN), 3,84 (s, 3H, CH₃), 4,49 (s, 2H, NH₂), 6,25 (s, 1H, H_{Ar}), 8,11 (s, 1H, H_{Ar}).
IR (KBr, cm⁻¹): 3453,8, 3342,7, 3209,3, 2952,1, 2927,6, 2860,0, 1621,7, 1577,9, 1468,1, 1450,2, 1412,1, 1262,9, 1216,4, 1177,1, 1040,6.

### Exemple 4

### 1-(4-amino-2-méthoxyphényl)-3-[1-(cyclohexylméthyl)-4-pipéridyl]propan-1-one

Le composé 4 est préparé suivant le mode opératoire de l'exemple 1 décrit ci-dessus en considérant les quantités suivantes :
4-[3-(4-amino-2-méthoxy)-3-oxopropyl]pipéridine-1-carboxylate de *tert*-butyle: 310 mg (0,86 mmol)
Acide trifluoroacétique : 2 ml
DCM : 2 ml
(iodométhyl)cyclohexane : 87 µL (0,63 mmol)
K₂CO₃ : 102 mg (0,74 mmol)
DMF : 5 ml
Le composé attendu est obtenu avec un rendement de 39% et se présente sous la forme d'une poudre jaune.
Il possède les caractéristiques suivantes :
PF = 90°C
C₂₂H₃₄N₂O₂
RMN ¹H (CDCl₃) : 0,87 (m, 2H, 2 CH), 1,18 (m, 6H, 6 CH), 1,47 (m, 1H, CH), 1,64 (m, 7H, 5 CH, CH₂), 1,75 (m, 2H, 2 CH), 1,81 (m, 2H, 2 CHN), 2,08 (d, J = 7,1Hz, 2H, CH₂N), 2,85 (m, 2H, 2 CHN), 2,90 (m, 2H, CH₂), 3,82 (s, 3H, CH₃), 4,09 (s, 2H, NH₂), 6,16 (d, J = 2,0Hz, 1H, H_{Ar}), 6,25 (dd, J = 8,5Hz, J = 2,0Hz, 1H, H_{Ar}), 7,70 (d, J = 8,5Hz, 1H, H_{Ar},).
IR (KBr, cm⁻¹) : 3447,7, 3366,5, 3245,1, 2922,8, 2847,0, 2809,3, 2770,5, 1590,7, 1601,8, 1508,7, 468,1, 1448,3, 1429,4, 1347,3, 1310;9, 1272,3, 1257,2, 1218,0, 1206,9, 1134,0, 1122,8, 1031,4, 823,1.

### Exemple 5

### 1-(4-amino-5-fluoro-2-méthoxyphényl)-3-[1-(cyclohexylméthyl)-4-pipéridyl]propan-1-one

A 72 mg de 4-[3-(4-amino-5-fluoro-2-méthoxy)-3-oxopropyl]pipéridine-1-carboxylate de *tert*-butyle (0,19 mmol) en solution dans 1 ml de DCM est ajouté 1 ml de TFA. Le milieu réactionnel est agité à TA pendant 15 minutes puis concentré à l'évaporateur rotatif sous pression réduite. Le résidu est dissous dans 3 ml de DMF auquel on ajoute 262 mg de K₂CO₃ (1,89 mmol) et 35 µL de iodométhylcyclohexane (0,25 mmol). Le nouveau milieu réactionnel est placé à 110°C pendant 3h. Après dilution dans l'AcOEt, le mélange est lavé 4 fois à la saumure, séché sur MgSO₄ puis concentré. Le résidu obtenu est purifié sur colonne de gel de silice (gradient d'élution : DCM/AcOEt 10/0 à 0/10 puis DCM + 2% Et₃N). Le produit attendu est obtenu avec un rendement de 56%.
Aspect : solide jaune
PF = 97 °C
C₂₂H₃₃FN₂O₂
RMN ¹H (CDCl₃) : 0,86 (m, 2H, 2 CH), 1,20 (m, 6H, 6 CH), 1,48 (m, 1H, CH), 1,64 (m, 7H, 5 CH, CH₂), 1,74 (m, 2H, 2 CH), 1,82 (m, 2H, 2 CHN), 2,08 (d, J = 7,1Hz, 2H, CH₂N), 2,85 (m, 2H, 2 CHN), 2,91 (m, 2H, CH₂), 3,84 (s, 3H, CH₃), 4,14 (s, 2H, NH₂), 6,27 (d, J_{H-F} = 7,1Hz, 1H, H_{Ar}), 7,55 (d, J_{H-F} = 12,2Hz, 1H, H_{Ar}).
IR (KBr, cm⁻¹) : 3352,5, 3220,5, 2921,4, 2849,5, 2801,2, 2768,3, 1626,5, 1601,8, 1520,8, 1467,2, 1449,7, 1428,3, 1357,0, 1310;9, 1250,5, 1207,2, 1171,0, 1148,3, 1028,9, 823,8.

### Exemple 6

### 1-(4-amino-5-chloro-2-méthoxyphényl)-3-[1-(cyclopropylméthyl)-4-pipéridyl]propan-1-one

Le composé 6 est préparé suivant le mode opératoire de l'exemple 5 décrit ci-dessus en considérant les quantités suivantes :
4-[3-(4-amino-5-chloro-2-méthoxy)-3-oxopropyl]pipéridine-1-carboxylate de *tert*-butyle :^{v} 159 mg (0,40 mmol)
Acide trifluoroacétique : 2 ml
DCM : 2 ml
(iodométhyl)cyclopropane : 43 mg (0,48 mmol)
K₂CO₃ : 553 mg (4,00 mmol)
DMF : 4 ml
Le composé attendu est obtenu avec un rendement de 39% et se présente sous la forme d'une poudre jaune.
PF = 162 °C
C₁₉H₂₇ClN₂O₂
RMN ¹H (CD₃OD) : 0,18 (m, 2H), 0,58 (m, 2H), 0,94 (m, 1H), 1,34 (m, 3H), 1,61 (m, 2H), 1,79 (m, 2H), 2,10 (m, 2H), 2,33 (m, 2H), 2,95 (m, 2H), 3,15 (m, 2H), 3,89 (s, 3H, CH₃), 6,31 (s, 1H, H_{Ar}), 7,51 (s, 1H, H_{Ar}).
IR (KBr, cm⁻¹) : 3466,5, 3349,8, 2923,7, 2849,4, 2804,8, 2779,7, 1644,0, 1621,5, 1586,4, 1500,8, 1465,2, 1419,9, 1376,9, 1313;2, 1252,0, 1215,3, 1177,9, 1118,6, 825,7.

### Exemple 7

### 1-(4-amino-5-chloro-2-méthoxyphényl)-3-[1-(cyclobutylméthyl)-4-pipéridyl]propan-1-one

Le composé 7 est préparé suivant le mode opératoire de l'exemple 5 décrit ci-dessus en considérant les quantités suivantes :
4-[3-(4-amino-5-chloro-2-méthoxy)-3-oxopropyl]pipéridine-1-carboxylate de *tert*-butyle : 159 mg (0,40 mmol)
Acide trifluoroacétique : 2 ml
DCM : 2 ml
(iodométhyl)cyclobutane : 34 µL (0,30 mmol)
K₂CO₃ : 45 mg (4,00 mmol)
DMF : 4 ml
Le composé attendu est obtenu avec un rendement de 45% et se présente sous la forme d'une poudre jaune.
PF = 143 °C
C₂₀H₂₉ClN₂O₂
RMN ¹H (CDCl₃) : 1,25 (m, 3H, 3 CH), 1,57 (m, 2H, CH₂), 1,78 (m, 8H, 4 CH_{BUT}, 2 CH, 2 CHN), 2,06 (m, 2H, 2 CH_{BUT}), 2,37 (d, J = 6,6Hz, 2H, CH₂N), 2,54 (m, 1H, CH_{BUT}), 2,83 (m, 2H, 2 CHN), 2,90 (m, 2H, CH₂), 3,85 (s, 3H, CH₃), 4,42 (s, 2H, NH₂), 6,25 (s, 1H, H_{Ar}), 7,78 (s, 1H, H_{Ar}).
IR (KBr, cm⁻¹) : 3484,7, 3226,9, 2923,5, 2852,3, 2804,8, 2763,1, 1642,3, 1623,5, 1574,6, 1453,8, 1420,7, 1397,2, 1306,3, 1257,4, 1214,1, 1175,4, 1011,2, 830,4.

### Exemple 8

### 1-(4-amino-5-chloro-2-méthoxyphényl)-3-[1-(cyclopentylméthyl)-4-pipéridyl]propan-1-one

Le composé 8 est préparé suivant le mode opératoire de l'exemple 1 décrit ci-dessus en considérant les quantités suivantes :
4-[3-(4-amino-5-chloro-2-méthoxy)-3-oxopropyl]pipéridine-1-carboxylate de *tert*-butyle : 134 mg (0,34 mmol)
Acide trifluoroacétique : 2 ml
DCM : 2 ml
(iodométhyl)cyclopentane : 57 mg (0,27 mmol)
K₂CO₃ : 41 mg (0,29 mmol)
DMF : 3 ml
Le composé attendu est obtenu avec un rendement de 35% et se présente sous la forme d'une poudre jaune.
PF = 142 °C
C₂₁H₃₁ClN₂O₂
RMN ¹H (CDCl₃) : 1,21 (m, 5H, 2 CH_{PENT}, 3 CH), 1,50 (m, 2H, 2 CH_{PENT}), 1,60 (m, 6H, 2 CH_{PENT}, CH₂, 2 CH), 1,76 (m, 2H, 2 CH_{PENT}), 1,86 (m, 2H, 2 CHN), 2,05 (m, 1H, CH_{PENT}), 2,24 (d, J = 7,1Hz, 2H, CH₂N), 2,90 (m, 4H, CH₂, 2 CHN), 3,85 (s, 3H, CH₃), 4,44 (s, 2H, NH₂), 6,26 (s, 1H, H_{Ar}), 7,79 (s, 1H, H_{Ar}).
IR (KBr, cm⁻¹) : 3485,6, 3332,1, 2936,2, 2923,8, 2860,0, 2799,7, 2768,0, 1643,8, 1622,7, 1599,5, 1574,2, 1453,4, 1421,6, 1310,5, 1266,0, 1214,2, 1175,8, 1018,4, 831,0.

### Exemple 9

### 1-(4-amino-5-chloro-2-méthoxyphényl)-3-[1-(cyclohexyl méthyl)-4-pipéridyl]propan-1-one

Le composé 9 est préparé suivant le mode opératoire de l'exemple 1 décrit ci-dessus en considérant les quantités suivantes :
4-[3-(4-amino-2-méthoxy)-3-oxopropyl]pipéridine-1-carboxylate de *tert*-butyle: 953 mg (2,41 mmol)
acide trifluoroacétique : 2 ml
DCM : 2 ml
(bromométhyl)cyclohexane : 262 µL (1,88 mmol)
K₂CO₃ : 281 mg (2,03 mmol)
DMF : 4 ml
Le composé attendu est obtenu avec un rendement de 44% présente sous la forme d'un solide jaune pâle.
Il possède les caractéristiques suivantes :
PF = 154°C
C₂₂H₃₃ClN₂O₂
RMN ¹H (CDCl₃) : 0,85 (m, 2H, 2 CH), 1,20 (m, 6H, 6 CH), 1,47 (m, 1H, CH), 1,68 (m, 11H, 7 CH, 2 CHN, CH₂), 2,08 (d, J = 6,8Hz, 2H, CH₂N), 2,84 (m, 2H, 2 CHN), 2,89 (m, 2H, CH₂), 3,85 (s, 3H, CH₃), 4,44 (s, 2H, NH₂), 6,26 (s, 1H, H_{Ar}), 7,79 (s, 1H, H_{Ar}).
IR (KBr, cm⁻¹) : 3484,8, 3229,8, 2920,7, 2850,0, 2803,0, 2768,0, 1640,7, 1623,0, 1575,0, 1452,8, 1420,9, 1313,8, 1255,6, 1214,3, 1177,2, 1012,7, 831,0.

### Exemple 10 (Non-couvert par l'invention)

### 1-(4-amino-5-chloro-2-méthoxyphényl)-3-[1-(cyclohexylméthyl)-4-pipéridyl]-2-méthylpropan-1-one

Le composé 10 est préparé suivant le mode opératoire de l'exemple 5 décrit ci-dessus en considérant les quantités suivantes :
4-[3-(4-amino-5-chloro-2-méthoxy)-2-méthyl-3-oxopropyl]pipéridine-1-carboxylate de *tert-*butyle : 50 mg (0,12 mmol)
Acide trifluoroacétique : 500 µL
DCM : 2 ml
(bromométhyl)cyclohexane : 20 µL (0,15 mmol)
K₂CO₃ : 168 mg (1,22 mmol)
DMF : 1 ml
Le composé attendu est obtenu avec un rendement de 42% et se présente sous la forme d'une huile jaune.
C₂₃H₃₅ClN₂O₂
RMN ¹H (CDCl₃) : 0,86 (m, 2H, 2 CH), 1,05 (d, J = 6,8Hz, 3H, CH₃), 1,19 (m, 6H, 6 CH), 1,49 (m, 1H, CH), 1,60 (m, 7H, 5 CH, CH₂), 1,73 (m, 2H, 2 CH), 1,86 (m, 2H, 2 CHN), 2,15 (d, J = 6,6Hz, 2H, CH₂N), 2,85 (m, 2H, 2 CHN), 3,57 (qd, J = 6,8Hz, 1H, CH), 3,81 (s, 3H, CH₃), 4,42 (s, 2H, NH₂), 6,24 (s, 1H, H_{Ar}), 7,67 (s, 1H, H_{Ar}).
IR (KBr, cm⁻¹) : 3477,5, 3351,5, 2922,4, 2851,8, 2801,6, 2766,2, 1650,4, 1621,4, 1590,8, 1464,5, 1450,3, 1417,3, 1305,1, 1255,8, 1214,8, 1178,2, 1007,6.

### Exemple 11

### 1-(4-amino-5-chloro-2-méthoxyphényl)-3-[1-[(2-méthylcyclohexyl)méthyl]-4-pipéridyl] propan-1-one

Le composé 11 est préparé suivant le mode opératoire de l'exemple 5 décrit ci-dessus en considérant les quantités suivantes :
4-[3-(4-amino-5-chloro-2-méthoxy)-3-oxopropyl]pipéridine-1-carboxylate de *tert*-butyle : 175 mg (0,44 mmol)
Acide trifluoroacétique : 1,5 ml
DCM : 3 ml
(iodométhyl)-2-méthylcyclohexane : 127 mg (0,53 mmol)
K₂CO₃ : 610 mg (4,42 mmol)
DMF : 4 ml
Le composé attendu est obtenu avec un rendement de 45% et se présente sous la forme d'un solide jaune.
PF = 111 °C
C₂₃H₃₅ClN₂O₂
RMN ¹H (CDCl₃) : 0,83 (d, J = 7,1Hz, 3H, CH₃), 1,28 (m, 7H, 7 CH), 1,60 (m, 8H, 6 CH, CH₂), 1,66 (m, 2H, 2 CH), 1,86 (m, 2H, 2 CHN), 2,13 (d, J = 6,8Hz, 2H, CH₂N), 2,89 (m, 4H, CH₂, 2 CHN), 3,84 (s, 3H, CH₃), 4,49 (s, 2H, NH₂), 6,27 (s, 1H, H_{Ar}), 7,78 (s, 1H, H_{Ar}).
IR (KBr, cm⁻¹): 3482,4, 3336,7, 3209,3, 2921,4, 2850,5, 2803,8, 2771,2, 1640,7, 1618,5, 1585,3, 1466,1, 1453,4, 1421,6, 1310,5, 1253,4, 1215,0, 1180,3, 1018,4.

### Exemple 12

### 1-(4-amino-5-chloro-2-méthoxyphényl)-3-[1-(cycloheptylméthyl)-4-pipéridyl]propan-1-one

Le composé 12 est préparé suivant le mode opératoire de l'exemple 5 décrit ci-dessus en considérant les quantités suivantes :
4-[3-(4-amino-5-chloro-2-méthoxy)-3-oxopropyl]pipéridine-1-carboxylate de *tert*-butyle : 152 mg (0,38 mmol)
Acide trifluoroacétique : 1 ml
DCM : 2 ml
(iodométhyl)cycloheptane : 110 mg (0,46 mmol)
K₂CO₃ : 535 mg (3,84 mmol)
DMF : 4 ml
Le composé attendu est obtenu avec un rendement de 42% et se présente sous la forme d'un solide jaune.
PF = 148 °C
C₂₃H₃₅ClN₂O₂
RMN ¹H (CDCl₃) : 1,12 (m, 2H, 2 CH), 1,50 (m, 18H, CH₂, 16 CH), 1,95 (m, 2H, 2 CHN), 2,15 (m, 2H, CH₂N), 2,89 (m, 4H, CH₂, 2 CHN), 3,85 (s, 3H, CH₃), 4,49 (s, 2H, NH₂), 6,27 (s, 1H, H_{Ar}), 7,78 (s, 1H, H_{Ar}).
IR (KBr, cm⁻¹) : 3481,0, 3247,7, 2919,6, 2850,2, 2800,8, 2768,1, 1637,6, 1621,7, 1582,3, 1453,6, 1419,5, 1307,1, 1252,8, 1214,4, 1176,1, 1012,6.

### Exemple 13

### Dichlorohydrate de 1-(4-amino-5-chloro-2-méthoxyphényl)-3-[1-[(pipéridin-4-yl)méthyl]-4-pipéridyl] propan-1-one

L'intermédiaire, menant au composé 13, est préparé suivant le mode opératoire de l'exemple 1 décrit ci-dessus en considérant les quantités suivantes :
4-[3-(4-amino-5-chloro-2-méthoxy)-3-oxopropyl]pipéridine-1-carboxylate de *tert*-butyle : 212 mg (0,53 mmol)
Acide trifluoroacétique : 1 ml
DCM : 2 ml
4-(iodométhyl)pipéridine-1-carboxylate de *tert*-butyle: 124 mg (0,38 mmol)
K₂CO₃ : 62 mg (0,45 mmol)
DMF : 4 ml
Cet intermédiaire est obtenu avec un rendement de 24% et se présente sous la forme d'une huile.
40 mg de cet intermédiaire (0,09 mmol) sont ensuite dissous dans 5 ml d'EtOH auxquels sont ajoutés 300 ml d'HCl concentré. Après 3 heures d'agitation à température ambiante, la solution est concentrée à l'évaporateur rotatif. Du toluène est ajouté pour faire un mélange azéotropique avec les traces d'eau restantes. Le résidu sec obtenu est ensuite repris dans l'éther éthylique puis filtré. 30 mg de produit attendu sont alors obtenus avec un rendement de 80%.
PF >260 °C
C₂₁H₃₄Cl₃N₃O₂
RMN ¹H (DMSO-d6) : 1,38 (m, 2H, 2 CH), 1,46 (m, 3H, CH, CH₂), 1,63 (m, 2H, 2 CH), 1,76 (m, 2H, 2 CH), 1,96 (m, 2H, 2 CH), 2,11 (m, 1H, CH), 2,81 (m, 6H, 4 CHN, CH₂), 2,91 (m, 2H, CH₂N), 3,22 (m, 2H, 2 CHN), 3,45 (m, 2H, 2 CHN), 3,79 (s, 3H, CH₃), 6,46 (s, 1H, H_{Ar}), 7,53 (s, 1H, H_{Ar}), 9,03 (m, 2H, 2 NH), 10,2 (m, 1H, NH).
IR (KBr, cm⁻¹): 3390,0, 3295,6, 3194,0, 2938,4, 2731,7, 1660,0, 1625,1, 1592,8, 1464,5, 1449,1, 1417,2, 1316,4, 1262,8, 1249,0, 1212,0, 1180,4.

### Exemple 14

### 1-(4-amino-5-bromo-2-méthoxyphényl)-3-[1-(cyclohexyl méthyl)-4-pipéridyl]propan-1-one

Le composé 14 est préparé suivant le mode opératoire de l'exemple 1 décrit ci-dessus en considérant les quantités suivantes :
4-[3-(4-amino-5-bromo-2-méthoxy)-3-oxopropyl]pipéridine-1-carboxylate de *tert*-butyle : 65 mg (0,19 mmol)
Acide trifluoroacétique : 1 ml
DCM : 1 ml
(bromométhyl)cyclohexane : 32 µL (0,23 mmol)
K₂CO₃ : 34 mg (0,25 mmol)
DMF : 4 ml
Le composé attendu est obtenu avec un rendement de 60% et se présente sous la forme d'une poudre jaune pâle.
PF = 148 °C
C₂₂H₃₃BrN₂O₂
RMN ¹H (CDCl₃) : 0,87 (m, 2H, 2 CH), 1,20 (m, 6H, 6 CH), 1,48 (m, 1H, CH), 1,60 (m, 7H, 5 CH, CH₂), 1,74 (m, 2H, 2 CH), 1,81 (m, 2H, 2 CHN), 2,09 (d, J = 6,8Hz, 2H, CH₂N), 2,87 (m, 4H, 2 CHN, CH₂), 3,84 (s, 3H, CH₃), 4,51 (s, 2H, NH₂), 6,26 (s, 1H, H_{Ar}), 7,93 (s, 1H, H_{Ar}).
IR (KBr, cm⁻¹): 3471,6, 3224,0, 2920,9, 2851,8, 2801,6, 2766,2, 1634,4, 1621,5, 1580,7, 1449,7, 1417,3, 1302;3, 1260,8, 1216,5, 1178,2, 1042,4.

### Exemple 15

### 1-(4-amino-5-iodo-2-méthoxyphényl)-3-[1-(cyclohexyl méthyl)-4-pipéridyl]propan-1-one

Le composé 12 est préparé suivant le mode opératoire de l'exemple 1 décrit ci-dessus en considérant les quantités suivantes :
4-[3-(4-amino-5-iodo-2-méthoxy)-3-oxopropyl]pipéridine-1-carboxylate de *tert*-butyle : 110 mg (0,23 mmol)
Acide trifluoroacétique : 2 ml
DCM : 2 ml
(bromométhyl)cyclohexane : 36 µL (0,26 mmol)
K₂CO₃ : 33 mg (0,24 mmol)
DMF : 2 ml
Le composé attendu est obtenu avec un rendement de 42% et se présente sous la forme d'une poudre jaune.
Il possède les caractéristiques suivantes :
PF = 178 - 180 °C
C₂₂H₃₃IN₂O₂
RMN ¹H (CDCl₃) : 0,85 (m, 2H, 2 CH), 1,20 (m, 6H, 6 CH), 1,47 (m, 1H, CH), 1,69 (m, 11H, 7 CH, 2 CHN, 1 CH₂), 2,07 (d, J = 7,1Hz, 2H, CH₂N), 2.84 (m, 2H, 2 CHN), 2,88 (m, 2H, CH₂), 3,84 (s, 3H, CH₃), 4,49 (s, 2H, NH₂), 6,25 (s, 1H, H_{Ar}), 8,11 (s, 1H, H_{Ar}).
IR (KBr, cm⁻¹): 3323,7, 3212,5, 2919,4, 2847,4, 2799,7, 2768,1, 1634,4, 1624,9, 1574,7, 1448,8, 1412,1, 1262,9, 1215,3, 1177,1, 1040,6, 834,2.

### Exemple 16

### 1-(4-amino-5-chloro-2-méthoxyphényl)-3-[1-(cyclohexyléthyl)-4-pipéridyl]propan-1-one

Le composé 16 est préparé suivant le mode opératoire de l'exemple 5décrit ci-dessus en considérant les quantités suivantes :
4-[3-(4-amino-5-chloro-2-méthoxy)-3-oxopropyl]pipéridine-1-carboxylate de *tert*-butyle :
145 mg (0,37 mmol)
Acide trifluoroacétique : 2 ml
DCM : 2 ml
(iodoéthyl)cyclohexane : 140 mg (0,44 mmol)
K₂CO₃ : 506 mg (3,66 mmol)
DMF : 5 ml
Le composé attendu est obtenu avec un rendement de 40% et se présente sous la forme d'une poudre jaune.
Il possède les caractéristiques suivantes :
PF=170°C
C₂₃H₃₅ClN₂O₂
RMN ¹H (CDCl₃) : 0,90 (m, 2H, 2 CH), 1,20 (m, 7H, 7 CH), 1,36 (m, 2H, CH₂), 1,61 (m, 9H, 7 CH, CH₂), 1,83 (m, 2H, 2 CHN), 2,28 (m, 2H, CH₂N), 2.89 (m, 4H, 2 CHN, CH₂), 3,82 (s, 3H, CH₃), 4,42 (s, 2H, NH₂), 6,23 (s, 1H, H_{Ar}), 7,76 (s, 1H, H_{Ar}).
IR (KBr, cm⁻¹): 3480,5, 2921,3, 2851,8, 1639,6, 1624,0, 1575,6, 1453,0, 1420,3, 1383,6, 1306,2, 1256,9, 1213,7, 1174,9, 1022,2, 830,2.

### Exemple 17 (Non-couvert par l'invention)

### 4-amino-5-chloro-N-[[1-(cyclohexylméthyl)-4-pipéridyl]méthyl]-2-méthoxybenzamide

Le composé 17 est préparé suivant le mode opératoire de l'exemple 5 décrit ci-dessus en considérant les quantités suivantes :
4-[[(4-amino-5-chloro-2-méthoxy-benzoyl)amino]méthyl]pipéridine-1-carboxylate de *tert-*butyle :^{vi} 66 mg (0,17 mmol)
Acide trifluoroacétique : 1 ml
DCM : 2 ml
(bromométhyl)cyclohexane : 39 mg (0,22 mmol)
K₂CO₃ : 232 mg (1,68 mmol)
DMF : 3 ml
Le composé attendu est obtenu avec un rendement de 38% et se présente sous la forme d'un solide blanc.
Il possède les caractéristiques suivantes :
PF = 154 - 155 °C
C₂₁H₃₂ClN₃O₂
RMN ¹H (CDCl₃) : 0,90 (m, 2H, 2 CH), 1,16 (m, 4H, 4 CH), 1,34 (m, 2H, 2 CH), 1,47 (m, 1H, CH), 1,68 (m, 7H, 7 CH), 1,88 (m, 2H, 2 CHN), 2,11 (d, J = 7,1Hz, 2H, CH₂N), 2,89 (m, 2H, 2 CHN), 3,29 (m, 2H, CH₂N), 3,88 (s, 3H, CH₃), 4,35 (s, 2H, NH₂), 6,27 (s, 1H, H_{Ar}), 7,73 (t, J = 5,2Hz, 1H, NH), 8,03 (s, 1H, H_{Ar}).
IR (KBr, cm⁻¹) : 3399,9, 2925,1, 2853,7, 1621,7, 1593,0, 1536,1, 1497,9, 1453,4, 1418,5, 1313,7, 1256,5, 1212,0, 1145,4, 989,8, 729,5.

### Exemple 18 (Non-couvert par l'invention)

### 4-amino-5-bromo-N-[[1-(cyclohexylméthyl)-4-pipéridyl]méthyl]-2-méthoxybenzamide

Le composé 18 est préparé suivant le mode opératoire de l'exemple 1 décrit ci-dessus en considérant les quantités suivantes :
4-[[(4-amino-5-bromo-2-méthoxy-benzoyl)amino]méthyl]pipéridine-1-carboxylate de *tert-*butyle : 250 mg (0,56 mmol)
Acide trifluoroacétique : 4 ml
DCM : 4 ml
(bromométhyl)cyclohexane : 68 µL (0,72 mmol)
K₂CO₃ : 92 mg (0,66 mmol)
DMF : 6 ml
PF = 162 °C
C₂₁H₃₂BrN₃O₂
RMN ¹H (CD₃OD) : 0,93 (m, 2H), 1,23 (m, 5H), 1,41 (m, 2H), 1,61 (m, 1H), 1,70 (m, 8H), 2,21 (m, 2H), 2,36 (m, 2H), 3.09 (m, 2H), 3,90 (s, 3H, CH₃), 6,27 (s, 1H, H_{Ar}), 7,94 (s, 1H, H_{Ar}).
IR (KBr, cm⁻¹) : 3450,5, 3406,0, 3319,5, 3195,6, 2918,3, 2847,5, 2763,4, 2417,3, 1634,8, 1593,8, 1543,1, 1496,2, 1463,1, 1448,0, 1309;8, 1255,6, 1210,2, 1179,1, 1134,8, 1041,2, 979,1, 838,9.

### Exemple 19 (Non-couvert par l'invention)

### 4-amino-5-iodo-N-[[1-(cyclohexylméthyl)-4-pipéridyl]méthyl]-2-méthoxybenzamide

Le composé 19 est préparé suivant le mode opératoire de l'exemple 5 décrit ci-dessus en considérant les quantités suivantes :
4-[[(4-amino-5-iodo-2-méthoxy-benzoyl)amino]méthyl]pipéridine-1-carboxylate de *tert-*butyle : 138 mg (0,28 mmol)
Acide trifluoroacétique : 1 ml
DCM : 2 ml
(bromométhyl)cyclohexane : 60 mg (0,34 mmol)
K₂CO₃ : 390 mg (2,82 mmol)
DMF : 3 ml
Le composé attendu est obtenu avec un rendement de 40% et se présente sous la forme d'une poudre jaune.
Il possède les caractéristiques suivantes :
PF=174°C
C₂₁H₃₂IN₃O₂
RMN ¹H (CDCl₃) : 0,85 (m, 2H, 2 CH), 1,20 (m, 4H, 4 CH), 1,34 (m, 2H, 2 CH), 1,47 (m, 1H, CH), 1,67 (m, 7H, 7 CH), 1,86 (m, 2H, 2 CHN), 2,10 (d, J = 7,1Hz, 2H, CH₂N), 2.88 (m, 2H, 2 CHN), 3.31 (m, 2H, CH₂N), 3,90 (s, 3H, CH₃), 4,40 (s, 2H, NH₂), 6,27 (s, 1H, H_{Ar}), 7,69 (t, J = 4,8Hz, 1H, NH), 8,45 (s, 1H, H_{Ar}).
IR (KBr, cm⁻¹) : 3451,5, 3407,6, 3313,5, 3196,4, 2916,7, 2845,4, 2792,0, 1636,2, 1582,8, 1551,4, 1490,2, 1446,1, 1408,6, 1302,1, 1267,0, 1211,3, 1182,3, 1143,0, 1043,1, 977,6, 836,4.

### Exemple 20 (Non-couvert par l'invention)

### [1-(cyclohexylméthyl)-4-pipéridyl]méthyl 4-amino-5-chloro-2-méthoxybenzoate

Sous N₂, à une suspension de 101 mg d'acide 4-amino-5-chloro-2-méthoxybenzoïque (0,50 mmol) dans 3 ml de THF fraîchement distillé sont ajoutés 89 mg de CDI (0,55 mmol). Le milieu réactionnel est agité à température ambiante pendant 24h puis une solution de 106 mg de (1-(cyclohexylméthyl)pipéridin-4-yl)méthanol (0,50 mmol) dans 2 ml de THF fraîchement distillé puis 21 mg de NaH (0,55 mmol). Après agitation à TA pendant un week-end, le THF est évaporé. Le résidu obtenu est repris dans l'AcOEt, lavé à l'eau puis séché sur MgSO₄. Après concentration, le brut réactionnel est purifié sur colonne de gel de silice (gradient d'élution : DCM 100% à AcOEt 100%) pour obtenir le produit attendu avec un rendement de 31%.
Aspect: solide blanc crème
PF = 129 °C
C₂₁H₃₁ClN₂O₃
RMN ¹H (CDCl₃) : 0,86 (m, 2H, 2 CH), 1,19 (m, 4H, 4 CH), 1,39 (m, 2H, 2 CH), 1,48 (m, 1H, CH), 1,69 (m, 7H, 7 CH), 1,86 (m, 2H, 2 CHN), 2,10 (d, J = 7,1Hz, 2H, CH₂N), 2,88 (m, 2H, 2 CHN), 3,84 (s, 3H, CH₃), 4,08 (d, J = 6,0Hz, 2H, CH₂O), 4,45 (s, 2H, NH₂), 6,29 (s, 1H, H_{Ar}), 7,81 (s, 1H, H_{Ar}).
IR (KBr, cm⁻¹) : 3472,9, 3329,8, 2919,7, 2850,5, 1694,9, 1621,3, 1600,1, 1449,3, 1316,9, 1275,6, 1234,3, 1109,2, 1072,4, 1053,3, 983,4.

### Exemple 21

### 1-(4-amino-5-chloro-2-hydroxyphényl)-3-[1-(cyclohexylméthyl)-4-pipéridyl]propan-1-one A une solution de 140 mg de 1-(4-amino-5-chloro-2-méthoxyphényl)-3-[1-(cyclohexylméthyl)-4-pipéridyl]propan-1-one (exemple 6, 0,36 mmol) dans 10 ml de CH₃CN

sont ajoutés 72 mg d'AlCl₃ (0,54 mmol) et 81 mg de NaI (0,54 mmol). Le milieu réactionnel est ensuite porté à reflux pendant 4h. Après dilution au DCM, la phase organique est lavée à l'eau puis au NaHCO₃ saturée. Après séchage sur MgSO₄, filtration et concentration, le résidu est purifié sur gel de silice (gradient d'élution : DCM à DCM/MeOH (9/1)) pour obtenir 109 mg de 1-(4-amino-5-chloro-2-hydroxyphényl)-3-[1-(cyclohexylméthyl)-4-pipéridyl]propan-1-one avec un rendement de 81%.
Aspect: solide jaune pâle
PF = 124 °C
C₂₁H₃₁ClN₂O₂
RMN ¹H (CDCl₃) : 0,86 (m, 2H, 2 CH), 1,22 (m, 6H, 6 CH), 1,48 (m, 1H, CH), 1,71 (m, 9H, 7 CH, CH₂), 1,85 (m, 2H, 2 CHN), 2,10 (d, J = 6,8Hz, 2H, CH₂N), 2,85 (m, 4H, CH₂, 2 CHN), 4,65 (s, 2H, NH₂), 6,23 (s, 1H, H_{Ar}), 7,61 (s, 1H, H_{Ar}), 12,72 (s, 1H, OH).
IR (KBr, cm⁻¹) : 3471,8, 3368,2, 2920,9, 2849,0, 2800,3, 2766,4, 1636,8, 1521,4, 1505,8, 1448,7, 1424,0, 1379,3, 1352,3, 1320,5, 1270,7, 1230,6, 1212,4, 1112,0, 812,5.

### Exemple 22

### 1-(4-amino-5-chloro-2-éthoxyphényl)-3-[1-(cyclohexyl méthyl)-4-pipéridyl]propan-1-one

A une solution de 37 mg de 1-(4-amino-5-chloro-2-hydroxyphényl)-3-[1-(cyclohexylméthyl)-4-pipéridyl]propan-1-one (0,10 mmol) dans 2 ml de DMF sont ajoutés 27 mg de K₂CO₃ (0,20 mmol) et 8,5 µL de iodoéthane (0,11 mmol). Le milieu réactionnel est agité 2h30 à 110°C. La solution est ensuite diluée à AcOEt puis est lavées 4 fois avec une solution NaCl saturée. La phase organique est ensuite séchée sur MgSO₄, filtrée et concentrée. Le résidu obtenu est ensuite purifié sur gel de silice (préparation : DCM + 2% Et₃N ; élution : DCM (9/1)) pour obtenir 33 mg de produit attendu (rdt = 84%) sous la forme d'un solide blanc.
PF = 147°C
C₂₃H₃₅ClN₂O₂
RMN ¹H (CDCl₃) : 0,81 (m, 2H, 2 CH), 1,20 (m, 6H, 6 CH), 1,44 (t, J = 7,1Hz, 3H, CH₃), 1,47 (m, 1H, CH), 1,68 (m, 11H, 7 CH, 2 CHN, CH₂), 2,05 (d, J = 6,8Hz, 2H, CH₂N), 2,82 (m, 2H, 2 CHN), 2,92 (t, J = 6,8Hz, 2H, CH₂), 4,03 (qd, J = 7,1Hz, 2H, CH₂O),4,39 (s, 2H, NH₂), 6,21 (s, 1H, H_{Ar}), 7,76 (s, 1H, H_{Ar}).
IR (KBr, cm⁻¹) : 3441,1, 3214,2, 2926,3, 2853,7, 1651,8, 1621,7, 1586,1, 1501,8, 1434,3, 1383,5, 1307,3, 1259,7, 1205,7, 1132,7, 1028,4, 802,5.

### Exemple 23

### 1-[4-amino-5-chloro-2-(2-fluoroéthoxy)phényl]-3-[1-(cyclohexylméthyl)-4-pipéridyl]propan-1-one

Le composé 23 est préparé suivant le mode opératoire de l'exemple 22 décrit ci-dessus en considérant les quantités suivantes :
1-(4-amino-5-chloro-2-hydroxyphényl)-3-[1-(cyclohexylméthyl)-4-pipéridyl]propan-1-one : 44 mg (0,12 mmol)
tosylate de 2-fluoroéthyle : 28 mg (0,13 mmol)
K₂CO₃ : 32 mg (0,23 mmol)
DMF : 3 ml
Le composé attendu est obtenu avec un rendement de 86% et se présente sous la forme d'un solide blanc.
Il possède les caractéristiques suivantes :
PF = 144-146 °C
C₂₃H₃₄ClFN₂O₂
RMN ¹H (CDCl₃) : 0,82 (m, 2H, 2 CH), 1,18 (m, 6H, 6 CH), 1,44 (m, 1H, CH), 1,66 (m, 11H, 7 CH, CH₂, 2 CHN), 2,05 (d, J = 7,1Hz, 2H, CH₂N), 2,81 (m, 2H, 2 CHN), 2,94 (m, 2H, CH₂), 4,17 (m, &H, CH), 4,24 (m, 1H, CH), 4,43 (s, 2H, NH₂), 4,69 (m, 1H, CH), 4,82 (m, 1H, CH), 6,20 (s, 1H, H_{Ar}), 7,78 (s, 1H, H_{Ar}).
IR (KBr, cm⁻¹) : 3431,6, 3266,5, 2926,7, 2853,7, 2675,9, 1644,3, 1618,5, 1586,8, 1501,0, 1450,2, 1434,3, 1383,5, 1342,3, 1310,5, 1256,5, 1205,7, 1183,5, 1164,4, 1072,4, 1047,3, 1018,4, 812,0.

### Exemple 24

### 2-chloro-4-[[2-[1-(cyclohexylméthyl)-4-pipéridyl]éthyl]-N-méthoxycarbonimidoyl]-5-méthoxyaniline

A une solution de 90 mg de 1-(4-amino-5-chloro-2-méthoxyphényl)-3-[1-(cyclohexylméthyl)-4-pipéridyl]propan-1-one (0,23 mmol) dans 2 ml de pyridine sont ajoutés 69 mg de chlorhydrate de méthoxyamine (0,82 mmol). Le milieu réactionnel est agité à température ambiante pendant 24h. Après dilution dans l'eau, la phase aqueuse est extraite 3 fois à l'AcOEt. Les phases organiques sont regroupées puis lavées 5 fois à la saumure, séchées sur MgSO₄ puis concentrées. Le résidu est purifié sur gel de silice (préparation : DCM + 2% Et₃N ; gradient d'élution DCM/AcOEt : 10/0 à 9/1) pour obtenir 41 mg du produit attendu (stéréoisomère E) sous la forme d'une huile jaune pâle (rdt = 42%). C₂₃H₃₆ClN₃O₂
RMN ¹H (CDCl₃) : 0,83 (m, 2H, 2 CH), 1,22 (m, 8H, 6 CH, CH₂), 1,44 (m, 1H, CH), 1,65 (m, 9H, 7 CH, 2 CHN), 2,04 (d, J = 6,8Hz, 2H, CH₂N), 2,63 (m, 2H, CH₂), 2,79 (m, 2H, 2 CHN), 3,72 (s,
3H, CH₃), 3,89 (s, 3H, CH₃), 4,09 (s, 2H, NH₂), 6,25 (s, 1H, H_{Ar}), 7,12 (s, 1H, H_{Ar}).
IR (KBr, cm⁻¹) : 3475,2, 3385,4, 2921,3, 2849,7, 2800,7, 2766,8, 1621,2, 1506,2, 1463,8, 1450,4, 1411,0, 1337,9, 1257,8, 1212,9, 1176,3, 1051,6, 984,8, 877,6.

### Exemple 25^{vii}

### 3-(2-méthoxy-4-méthylphényl)-3-oxopropanoate d'éthyle

A une suspension de 300 mg d'acide 4-amino-2-méthoxybenzoïque (1,78 mmol) dans 15 ml de THF distillé sont additionnés précautionneusement 320 mg de CDI (1,98 mmol). Le mélange est agité à température ambiante pendant 4h. Ensuite, sont ajoutés par portion 366 mg du sel potassique du 3-éthoxy-3-oxopropanoate (2,16 mmol) et 205 mg de MgCl₂ (2,16 mmol). Le mélange réactionnel est agité à température ambiante pendant 2 jours. Après extension avec 30 ml d'Et₂O, la solution est lavée successivement à l'eau, à une solution saturée de NaHCO₃ et à une solution saturée de NaCl. Après séchage sur MgSO₄, la phase organique est évaporée et le produit brut est purifié sur gel de silice (gradient d'élution : DCM à DCM/AcOEt 95/5) pour donner 194 mg de 3-(4-amino-2-méthoxyphényl)-3-oxopropanoate d'éthyle avec un rendement de 46%.
Aspect : huile incolore
C₁₂H₁₅NO₄
RMN ¹H (CDCl₃) : 1,24 (t, J = 7,8Hz, 3H, CH₃), 3,82 (s, 3H, CH₃), 3,88 (s, 2H, CH₂), 4,17 (q, J = 7,8Hz, 2H, CH₂), 4,18 (s, 2H, NH₂), 6,12 (d, J = 2,1Hz, 1H, H_{Ar}), 6,27 (dd, J = 8,8Hz, J = 2,1Hz, 1H, H_{Ar}), 7,83 (d, J = 8,8Hz, 1H, H_{Ar}).
IR (KBr, cm⁻¹) : 3460,2, 3365,2, 3228,0, 2980,7, 1729,3, 1640,7, 1592,1, 1471,0, 1433,6, 1322,6, 1253,2, 1215,2, 1129,9, 1026,2, 830,3.

### Exemple 26¹⁰

### 3-(4-amino-5-chloro-2-méthoxy-phényl)-3-oxopropanoate d'éthyle

A une suspension de 603 mg d'acide 4-amino-5-chloro-2-méthoxybenzoïque (2,99 mmol) dans 30 ml de THF distillé sont additionnés précautionneusement 533 mg de CDI (3,29 mmol).¹⁰ Le mélange est agité à température ambiante pendant 6h. Ensuite, sont ajoutés par portion 611 mg du sel potassique du 3-éthoxy-3-oxopropanoate (3,59 mmol) et 342 mg de MgCl₂ (3,59 mmol). Le mélange réactionnel est agité à 40°C pendant 2 jours. Après extension avec 30 ml d'Et₂O, la solution est lavée successivement à l'eau, à une solution saturée de NaHCO₃ et à une solution saturée de NaCl. Après séchage sur MgSO₄, la phase organique est évaporée et le produit brut est purifié sur gel de silice (gradient d'élution : CH/AcOEt 8/2 à 7/3) pour donner 507 mg de 3-(4-amino-5-chloro-2-méthoxyphényl)-3-oxopropanoate d'éthyle avec un rendement de 62%.
Aspect: solide blanc
PF = 124 °C
C₁₂H₁₄ClNO₄
RMN ¹H (CDCl₃) : 1,24 (t, J = 7,3Hz, 3H, CH₃), 3,82 (s, 3H, CH₃), 3,87 (s, 2H, CH₂), 4,17 (q, J = 7,3Hz, 2H, CH₂), 4,56 (s, 2H, NH₂), 6,23 (s, 1H, H_{Ar}), 7,92 (s, 1H, H_{Ar}).
IR (KBr, cm⁻¹) : 3463,0, 3362,5, 3222,9, 2982,6, 1725,0, 1647,9, 1621,7, 1572,8, 1468,9, 1456,6, 1422,8, 1325,5, 1261,6, 1221,0, 1155,4, 1024,0, 836,8.

### Exemple 27

### 3-(4-amino-5-fluoro-2-méthoxy-phényl)-3-oxopropanoate d'éthyle

Le composé 27 est préparé suivant le mode opératoire de l'exemple 26 décrit ci-dessus en considérant les quantités suivantes :
Acide 4-amino-5-fluoro-2-méthoxybenzoïque : 272 mg (1,47 mmol)
CDI : 262 mg (1,62 mmol)
sel potassique du 3-éthoxy-3-oxopropanoate : 300 mg (1,76 mmol)
MgCl₂ : 168 mg (1,76 mmol)
THF : 30 ml
Le composé attendu est obtenu avec un rendement de 65% et se présente sous la forme d'un solide blanc.
PF = 83-85 °C
C₁₂H₁₄FNO₄
RMN ¹H (CDCl₃) : 1,24 (t, J = 7,1Hz, 3H, CH₃), 3,82 (s, 3H, CH₃), 3,88 (s, 2H, CH₂), 4,17 (q, J = 7,1Hz, 2H, CH₂), 4,26 (s, 2H, NH₂), 6,24 (d, J = 7,1Hz, 1H, H_{Ar}), 7,65 (d, J = 11,9Hz, 1H, H_{Ar}).
IR (KBr, cm⁻¹) : 3469,7, 3464,2, 3231,6, 2981,7, 1729,6, 1652,9, 1628,0, 1604,2, 1522,4, 1469,6, 1431,7, 1366,9, 1319,2, 1250,2, 1211,3, 1189,8, 1139,2, 1026,3, 829,9.

### Exemple 28

### 3-(4-amino-5-bromo-2-méthoxy-phényl)-3-oxopropanoate d'éthyle

Le composé 28 est préparé suivant le mode opératoire de l'exemple 26 décrit ci-dessus en considérant les quantités suivantes :
Acide 4-amino-5-bromo-2-méthoxybenzoïque : 500 mg (2,03 mmol)
CDI : 362 mg (2,23 mmol)
sel potassique du 3-éthoxy-3-oxopropanoate : 414 mg (2,44 mmol)
MgCl₂ : 232 mg (2,44 mmol)
THF : 30 ml
Le composé attendu est obtenu avec un rendement de 50% et se présente sous la forme d'une poudre blanche.
Il possède les caractéristiques suivantes :
PF = 128 °C
C₁₂H₁₄BrNO₄
RMN ¹H (CDCl₃) : 1,24 (t, J = 7,8Hz, 3H, CH₃), 3,83 (s, 3H, CH₃), 3,87 (s, 2H, CH₂), 4,18 (q, J = 7,8Hz, 2H, CH₂), 4,59 (s, 2H, NH₂), 6,23 (s, 1H, H_{Ar}), 8,08 (s, 1H, H_{Ar}).
IR (KBr, cm⁻¹) : 3456,3, 3358,0, 3217,6, 2981,0, 1724,9, 1647,4, 1620,4, 1578,0, 1468,2, 1454,3, 1420,4, 1321,5, 1265,7, 1221,2, 1153,7, 1023,7, 835,0.

### Exemple 29

### 3-(4-amino-5-iodo-2-méthoxy-phényl)-3-oxopropanoate d'éthyle

Le composé 29 est préparé suivant le mode opératoire de l'exemple 26 décrit ci-dessus en considérant les quantités suivantes :
Acide 4-amino-5-iodo-2-méthoxybenzoïque : 355 mg (1,21 mmol)
CDI : 216 mg (1,33 mmol)
sel potassique du 3-éthoxy-3-oxopropanoate : 247 mg (1,45 mmol)
MgCl₂ : 138 mg (1,45 mmol)
THF : 30 ml
Le composé attendu est obtenu avec un rendement de 55% et se présente sous la forme d'une poudre blanche.
Il possède les caractéristiques suivantes :
PF = 140 °C
C₁₂H₁₄INO₄
RMN ¹H (CDCl₃) : 1,24 (t, J = 7,8Hz, 3H, CH₃), 3,82 (s, 3H, CH₃), 3,87 (s, 2H, CH₂), 4,17 (q, J = 7,8Hz, 2H, CH₂), 4,65 (s, 2H, NH₂), 6,22 (s, 1H, H_{Ar}), 8,25 (s, 1H, H_{Ar}).
IR (KBr, cm⁻¹) : 3454,0, 3350,0, 3212,1, 2976,2, 1732,2, 1645,6, 1619,0, 1569,6, 1467,0, 1454,5, 1421,4, 1325,2, 1264,7, 1225,9, 1190,5, 1153,4, 1031,9, 831,2.

### Exemple 30^{viii}

### 4-[3-(4-amino-2-méthoxyphényl)-3-oxopropyl]pipéridine-1-carboxylate de tert-butyle

A une solution de 379 mg du composé de l'exemple 22 (1,58 mmol) dans 30 ml de DMF sont ajoutés 437 mg de K₂CO₃ (3,16 mmol) et 617 mg de 4-(iodométhyl)pipéridine-1-carboxylate de *tert*-butyle (1,89 mmol). Le mélange réactionnel est agité à température ambiante pendant 1 semaine. Après dilution dans l'eau, le produit est extrait à l'Et₂O (3 X 40 ml). Les phases organiques sont regroupées, lavées 3 fois à l'eau et une fois à la saumure puis séchées sur MgSO₄. Après concentration, le résidu est dissous dans 10 ml d'un mélange binaire EtOH/H₂O (5/1) auquel 407 mg de KOH (7,27 mmol) sont ajoutés. Le nouveau milieu réactionnel est porté à reflux pendant 4 heures. L'EtOH est éliminé à l'évaporateur rotatif puis la phase aqueuse résultante est diluée à l'eau, extraite au DCM. Les phases organiques sont regroupées, lavées à l'eau et à la saumure, séchées sur MgSO₄ puis concentrées. Le produit brut est purifié sur gel de silice (gradient d'élution : DCM à DCM/AcOEt 6/4) pour obtenir 469 mg du 4-[3-(4-amino-2-méthoxy)-3-oxopropyl]pipéridine-1-carboxylate de *tert*-butyle avec un rendement de 82%.

Aspect: huile jaune
C₂₀H₃₀N₂O₄
RMN ¹H (CDCl₃) : 1,11 (m, 2H, 2 CH), 1,45 (m, 10H, 3 CH₃, CH), 1,61 (m, 2H, CH₂), 1,68 (m, 2H, 2 CH), 2,67 (m, 2H, 2 CHN), 2,90 (m, 2H, CH₂), 3,86 (s, 3H, CH₃), 4,07 (m, ,2H, 2 CHN), 4,62 (s, 2H, NH₂), 6,16 (d, J = 2,0Hz, 1H, H_{Ar}), 6,27 (dd, J = 8,6Hz, J = 2,0Hz, 1H, H_{Ar}), 7,71 (d, J = 8,6Hz, 1H, H_{Ar}).
IR (KBr, cm⁻¹) : 3442,8, 3355,8, 3240,2, 2974,2, 2930,5, 2851,3, 1675,9, 1642,3, 1595,9, 1468,4, 1431,1, 1365,6, 1277,7, 1212,9, 1162,6.

### Exemple 31

### 4-[3-(4-amino-5-chloro-2-méthoxyphényl)-2-méthyl-3-oxopropyl]pipéridine-1-carboxylate de tert-butyle

A une solution de 300 mg de 4-[3-(4-amino-5-chloro-2-méthoxyphényl)-2-méthyl-3-oxopropyl]pipéridine-1-carboxylate de *tert*-butyle (0,76 mmol) dans 3 ml de THF fraîchement distillé sont ajoutés, sous N₂, à -10°C, 909 µL de LIHMDS 1M dans le THF (0,909 mmol). Le milieu réactionnel est agité 15 minutes à -10°C puis sont additionnés 57 ml de iodométhane (0,909 mmol). Après 3h et un retour tranquille à TA, la solution est concentrée sous pression réduite. Le résidu est repris dans l'AcOEt et lavée 2 fois à l'eau. La phase organique est séchée sur MgSO₄ puis concentrées. Le produit brut est purifié sur gel de silice (gradient d'élution : DCM à DCM/AcOEt 95/5) pour obtenir 76 mg de produit attendu (rdt = 25%).

Aspect: huile jaune pâle
C₂₁H₃₁ClN₂O₄
RMN ¹H (CDCl₃) : 1,00 (m, 2H, 2 CH), 1,06 (d, J = 7,1Hz, 3H, CH₃) 1,18 (m, 1H, 1 CH), 1,46 (m, 10H, 3 CH₃, CH), 1,55 (m, 2H, 2 CH), 1,68 (m, 1H, 1 CH), 2,62 (m, 2H, 2 CHN), 2,90 (m, 2H, 2 CHN), 3,58 (sext, J = 7,1Hz, 1H, CH), 3,81 (s, 3H, CH₃), 4,01 (m, ,2H, 2 CHN), 4,44 (s, 2H, NH₂), 6,24 (s, 1H, H_{Ar}), 7,67 (s, 1H, H_{Ar}).
IR (KBr, cm⁻¹) : 3471,8, 3350,1, 3240,2, 2973,9, 2927,8, 2849,4, 1673,2, 1622,4, 1590,1, 1465,1, 1419,2, 1365,5, 1278,3, 1250,5, 1215,6, 1174,8.

### Exemple 32

### 4-[3-(4-amino-5-fluoro-2-méthoxyphényl)-3-oxopropyl]pipéridine-1-carboxylate de tert-butyle

Le composé 32 est préparé suivant le mode opératoire de l'exemple 30 décrit ci-dessus en considérant les quantités suivantes :
3-(4-amino-5-fluoro-2-méthoxyphényl)-3-oxopropanoate d'éthyle: 185 mg (0,72 mmol)
4-(iodométhyl)pipéridine-1-carboxylate de *tert*-butyle : 283 mg (0,87 mmol)
K₂CO₃ : 200 mg (1,45 mmol)
DMF : 5 ml
KOH : 187 mg (3,33 mmol)
EtOH :10 ml
H₂O : 2 ml
Le composé attendu est obtenu avec un rendement de 70% et se présente sous la forme d'une poudre blanche.
Il possède les caractéristiques suivantes :
PF = 171°C
C₂₀H₂₉FN₂O₄
RMN ¹H (CDCl₃) : 1,11 (m, 2H, 2 CH), 1,43 (m, 1H, CH), 1,45 (s, 9H, 3 CH₃), 1,59 (m, 2H, CH₂), 1,67 (m, 2H, 2 CH), 2,67 (m, 2H, 2 CH), 2,92 (m, 2H, CH₂), 3,84 (s, 3H, CH₃), 4,08 (m, ,2H, 2 CHN), 4,15 (s, 2H, NH₂), 6,27 (d, J_{H-F} = 7.1Hz, 1H, H_{Ar}), 7,94 (s, J_{H-F} = 11.9Hz, 1H, H_{Ar}).
IR (KBr, cm⁻¹) : 3438,0, 3350,6, 3229,9, 2974,2, 2927,4, 2853,0, 1675,1, 1628,6, 1604,4, 1467,6, 1428,0, 1365,6, 1311,5, 1249,4, 1168,8.

### Exemple 33

### 4-[3-(4-amino-5-bromo-2-méthoxyphényl)-3-oxopropyl]pipéridine-1-carboxylate de tert-butyle

Le composé 33 est préparé suivant le mode opératoire de l'exemple 30 décrit ci-dessus en considérant les quantités suivantes :
3-(4-amino-5-bromo-2-méthoxyphényl)-3-oxopropanoate d'éthyle : 250 mg (0,79 mmol) 4-(iodométhyl)pipéridine-1-carboxylate de *tert*-butyle : 309 mg (0,95 mmol)
K₂CO₃ : 218 mg (158 mmol)
DMF : 5 ml
KOH : 204 mg (3,63 mmol)
EtOH : 8 ml
H₂O : 2 ml
Le composé attendu est obtenu avec un rendement de 68% et se présente sous la forme d'une poudre blanche.
Il possède les caractéristiques suivantes :
PF = 146-148°C
C₂₀H₂₉BrN₂O₄
RMN ¹H (CDCl₃) : 1,07 (m, 2H, 2 CH), 1,43 (m, 10H, 3 CH₃, CH), 1,57 (m, 2H, CH₂), 1,64 (m, 2H, 2 CH), 2,64 (m, 2H, 2 CH), 2,88 (m, 2H, CH₂), 3,81 (s, 3H, CH₃), 4,06 (m, ,2H, 2 CHN), 4,61 (s, 2H, NH₂), 6,25 (s, 1H, H_{Ar}), 7,91 (s, 1H, H_{Ar}).
IR (KBr, cm⁻¹) : 3469,7, 3353,1, 3225,2, 2977,5, 2928,4, 2853,7, 1688,8, 1672,5, 1621,6, 1584,9, 1419,6, 1365,8, 1311,6, 1250,4, 1218,0, 1172,8.

### Exemple 34

### 4-[3-(4-amino-5-iodo-2-méthoxyphényl)-3-oxopropyl]pipéridine-1-carboxylate de tert-butyle

Le composé 33 est préparé suivant le mode opératoire de l'exemple 30 décrit ci-dessus en considérant les quantités suivantes :
3-(4-amino-5-iodo-2-méthoxyphényl)-3-oxopropanoate d'éthyle : 172 mg (0,47 mmol) 4-(iodomethyl)piperidine-1-carboxylate de *tert*-butyle : 169 mg (0,52 mmol)
K₂CO₃ : 131 mg (0,95 mmol)
DMF : 5 ml
KOH : 122 mg (2,18 mmol)
EtOH: 8 ml
H₂O: 2 ml
Le composé attendu est obtenu avec un rendement de 65% et se présente sous la forme d'une poudre blanche.
Il possède les caractéristiques suivantes :
PF = 152°C
C₂₀H₂₉IN₂O₄
RMN ¹H (CDCl₃) : 1,10 (m, 2H, 2 CH), 1,45 (m, 10H, 3 CH₃, CH), 1,59 (m, 2H, CH₂), 1,67 (m, 2H, 2 CH), 2,67 (m, 2H, 2 CHN), 2,90 (m, 2H, CH₂), 3,85 (s, 3H, CH₃), 4,08 (m, ,2H, 2 CHN), 4,51 (s, 2H, NH₂), 6,25 (s, 1H, H_{Ar}), 8,13 (s, 1H, H_{Ar}).
IR (KBr, cm⁻¹) : 3459,4, 3341,2, 3219,9, 2972,8, 2928,5, 2850,1, 1672,7, 1621,3, 1578,9, 1451,4, 1415,0, 1365,2, 1265,5, 1217,3, 1164,6.

### Exemple 35

### 4-[[(4-amino-5-bromo-2-méthoxybenzoyl)amino]méthyl]pipéridine-1-carboxylate de tert-butyle

A une solution de 474 mg d'acide 4-amino-5-bromo-2-méthoxybenzoïque (1,93 mmol) dans 4 ml de DMF sont ajoutés à 269 ml de Et₃N (1,93 mmol) et 414 mg de 4-(aminométhyl)pipéridine -1-carboxylate de *tert*-butyle (1,93 mmol).⁹ La solution est placée à -5°C puis sont ajoutés 261 mg de HOBT (1,93 mmol) et 371 mg d'EDCI.HCl (1,93 mmol). Le milieu réactionnel est ensuite ajouté une nuit à TA. La solution est diluée à l'eau puis extraite 3 fois à l'AcOEt. Les phases organiques sont regroupées puis lavées 4 fois à l'eau . La phase organique est séchée sur MgSO₄, filtrée puis concentrée. Le brut réactionnel est ensuite purifié sur gel de silice (gradient d'élution : DCM à DCM/AcOEt 7/3) pour obtenir 630 mg de produit attendu (rdt = 74%).
PF = 125°C
C₁₉H₂₈BrN₃O₄RMN ¹H (CDCl₃) : 1,17 (m, 2H, 2 CH), 1,45 (m, 9H, 3 CH₃), 1,75 (m, 3H, 3 CH), 2,69 (m, 2H, 2 CHN), 3,32 (m, 2H, CH₂N), 3,89 (s, 3H, CH₃), 4,10 (m, ,2H, 2 CHN), 4,53 (s, 2H, NH₂), 6,32 (s, 1H, H_{Ar}), 7,76 (t, J = 5,6Hz, 1H, NH), 8,24 (s, 1H, H_{Ar}).
IR (KBr, cm⁻¹) : 3448,2, 3337,2, 3206,8, 2970,9, 2927,7, 2854,9, 1689,0, 1631,6, 1593,5, 1559,2, 1491,2, 1465,4, 1430,2, 1364,5, 1247,0, 1179,2, 1147,3.

### Exemple 36

### 4-[[(4-amino-5-iodo-2-méthoxybenzoyl)amino]méthyl]pipéridine-1-carboxylate de tert-butyle

Le composé 35 est préparé suivant le mode opératoire de l'exemple 34 décrit ci-dessus en considérant les quantités suivantes :
acide 4-amino-5-iodo-2-méthoxybenzoïque : 342 mg (1,16 mmol)
4-(aminométhyl)pipéridine-1-carboxylate de *tert*-butyle : 214 mg (1,16 mmol)
Et₃N : 162 µL (1,16 mmol)
EDCI.HCl : 223 mg (1,16 mmol)
HOBT: 157 mg (1,16 mmol)
DMF : 5 ml
Le composé attendu est obtenu avec un rendement de 80% et se présente sous la forme d'une poudre jaune pâle.
Il possède les caractéristiques suivantes :
PF = 135°C
C₁₉H₂₈IN₃O₄
RMN ¹H (CDCl₃) : 1,18 (m, 2H, 2 CH), 1,45 (m, 9H, 3 CH₃), 1,73 (m, 3H, 3 CH), 2,69 (m, 2H, 2 CHN), 3,32 (m, 2H, CH₂N), 3,90 (s, 3H, CH₃), 4,12 (m, ,2H, 2 CHN), 4,42 (s, 2H, NH₂), 6,28 (s, 1H, H_{Ar}), 7,72 (t, J = 5,8Hz, 1H, NH), 8,45 (s, 1H, H_{Ar}).
IR (KBr, cm⁻¹) : 3408,0, 3333,5, 3209,0, 2973,8, 2927,4, 2850,1, 1679,3, 1627,9, 1586,5, 1536,2, 1491,2, 1465,4, 1425,6, 1365,3, 1251,7, 1212,4, 1171,3, 1142,5.

Les propriétés biologiques des composés selon l'invention mentionnés ci-dessus ont été testées afin de déterminer :
- leur affinité vis-à-vis du récepteur 5-HT4
- la puissance de leur effet agoniste vis-à-vis du récepteur 5-HT4
- leur effet inhibiteur de l'acétylcholinestérase (test d'Ellman)
- leur interaction avec le site périphérique de l'acétylcholinestérase (test de déplacement du propidium)
- leur capacité à augmenter in vivo chez le rongeur le délai d'extinction de la trace mnésique dans un test de reconnaissance d'objets modélisant la mémoire épisodique (nature de l'objet) chez le rongeur (test de reconnaissance d'objet).

### A) affinité vis-à-vis des récepteurs 5-HT4

### Matériel et méthodes

### Prélèvement du tissu striatal et préparations membranaires

L'ensemble des procédures décrites dans ce chapitre est issu des travaux de Grossman et al. (1993). Brièvement, les animaux (Cobayes mâles : 300-350 g, IFFA CREDO, France) sont euthanasiés par décapitation, puis le cerveau rapidement prélevé à +4°C. Les régions striatales sont soigneusement disséquées puis regroupées. L'ensemble des striata est placé dans 10 volumes de tampon HEPES 50mM, pH 7,4 à +4°C. Après homogénéisation à +4°C (Ultra-Turrax, vitesse maximale, 15 sec), et ultracentrifugation (23 000 g, 60 min, +4°C), le culot est remis en suspension dans du tampon HEPES 50mM, pH 7,4 à +4°C de manière à obtenir une concentration tissulaire de l'ordre de 15 mg.ml⁻¹ (dosage des protéines par la méthode de Lowry et al., 1951, utilisant l'albumine de sérum bovin comme standard).

### Etudes de compétition des produits à étudier en compétition contre le [³H]-GR113808

Préalablement aux études de compétition, une série de courbes de saturation sont effectuées afin de vérifier que les paramètres pharmacologiques Kd, Bmax et coefficient de Hill du [³H]-GR113808, obtenus dans nos conditions expérimentales, sont concordants avec ceux publiés dans la littérature. Pour cela, des échantillons de préparation membranaire à 7,5 µg/µL, sont incubés en duplicata (tampon HEPES 50 mM, pH 7.4, +37°C) à +37°C et pendant 30 minutes, en présence de 7 concentrations croissantes (0,05-1,5 nM, 200 µl de volume final) de [3H]-GR113808 (Grossman et al., 1993). Au moyen d'un système Brandel Cell Harvester, la liaison du radioligand aux protéines est interrompue par filtration rapide du milieu d'incubation sur une bande filtre (FP-100 WHATMAN GF/B) pré-incubée dans la polyéthylénimine (PEI 0,5% dans l'eau) ; la filtration est suivie de 3 rinçages par 4 ml de tampon HEPES 50 mM, pH 7,4 à +4°C. La liaison non spécifique du [³H]-GR113808 est quantifiée en présence de 30 µM de sérotonine, la liaison spécifique étant ainsi estimée comme la différence : (liaison en absence de sérotonine)-(liaison en présence de sérotonine).

Les études de compétition sont effectuées avec 0,1 nM de [³H]-GR113808 en présence de 10⁻⁶ ou 10⁻⁸ M, ou n concentrations pour les Ki, de ligand à étudier, le prucalopride étant utilisé comme agoniste 5-HT4 de référence. Après incubation, les procédures de filtration et de rinçage étaient identiques à celles décrites ci-dessus.

### Résultats

A titre d'exemples les affinités de plusieurs composés selon l'invention vis-à-vis des récepteurs 5-HT4 sont reportées dans le tableau 4.

**Tableau 4 : affinité de plusieurs composés selon l'invention vis-à-vis des récepteurs 5-HT4, le prucalopride étant pris comme référence (Ki en nM).**

| composé | Ki en nM |
|---|---|
| **1*** | 88,6 |
| **2*** | 8,9 |
| **3*** | 44,7 |
| **4** | 125,0 |
| **5** | 3,8 |
| **6** | 14,8 |
| **7** | 8,8 |
| **8** | 3,0 |
| **9** | 7,1 |
| **10** | 125,0 |
| **11** | 8,0 |
| **12** | 3,9 |
| **13** | 2,5 |
| **14** | 17,0 |
| **15** | 18,1 |
| **16** | 8,2 |
| **17 *** | 2,3 |
| **18 *** | 5,4 |
| **19 *** | 13,9 |
| **20 *** | 0,9 |
| **21** | 125,0 |
| **22** | 12,5 |
| **23** | 2,5 |
| **24** | 13,1 |
| **prucalopride** | 44,1 |

| | |
|---|---|
| * : Non-couvert par l'invention | |

### B) caractère agoniste vis-à-vis du récepteur 5-HT4

### Matériel et méthodes

Le caractère agoniste vis-à-vis du récepteur 5-HT4 de plusieurs composés selon l'invention est déterminé par la mesure de l'accumulation d'AMP cyclique intracellulaire. Pour cela, des cellules stables transfectées sont cultivées jusqu'à confluence et incubées avec un milieu serum-free 4 h avant le début de l'expérimentation. Les cellules sont ensuite pré-incubées durant 15 min avec un milieu serum-free supplémenté avec 5 mM de théophylline, 10 µM de pargyline et 1 µM de composé GR127935 dans des cellules CHO pour bloquer l'activité endogène des récepteurs 5-HT1B. De la sérotonine est ensuite ajoutée pendant 15 minutes supplémentaires. La réaction est stoppée par aspiration du milieu et addition de 500 µL d'éthanol glacé. Après 30 min d'incubation, la fraction éthanolique est collectée et évaporée sous vide. La pastille est reconstituée et l'AMP cyclique est quantifié par test radioimmunologique (cyclic AMP competitive radioimmunoassay, Immunotech, Marseille, France). Les tests de Student sont réalisés en utilisant le logiciel QuickTTest. Les résultats sont exprimés en pourcentage d'effet agoniste par rapport à la sérotonine. Le prucalopride, agoniste entier des récepteurs 5-HT4 est utilisé comme référence.

### Résultats

A titre d'exemples les puissances d'effet agoniste de plusieurs composés selon l'invention vis-à-vis des récepteurs 5-HT4 sont reportées dans le tableau 5.

**Tableau 5 : puissance de l'effet agoniste de plusieurs composés selon l'invention vis-à-vis des récepteurs 5-HT4 (%).**

| composé | % effet agoniste |
|---|---|
| **5** | 16% |
| **8** | 12% |
| **9** | 22% |
| **12** | 21% |
| **prucalopride** | 100% |

### C) Test d'Ellman

### Matériel et méthodes

De l'acétylcholinestérase extraite d'érythrocytes humains (solution aqueuse tamponnée, ≥ 500 unités/mg, Sigma Aldrich) est diluée dans 20mM de tampon HEPES à pH8, 0,1% Triton X-100 de façon à obtenir d'une solution titrée à 2,5 unités d'activité enzymatique / ml. 100 µL d'une solution d'acide 5,5-dithiobis(2-nitrobenzoïque) (DTNB) à 0,3mM dans un tampon phosphate à pH7,4 sont introduits dans une plaque 96 puits, suivis de 50µL de composé à tester en solution dans le DMSO et de 50µL de solution d'enzyme. Après 5 min de préincubation, la réaction est initiée par l'injection de 50µL de solution à 10mM d'iodure d'acétylthiocholine. L'hydrolyse de l'acétylthiocholine est suivie par la formation de l'anion 5-thio-2-nitrobenzoate, produit de la réaction du DTNB avec la thiocholine libérée par l'hydrolyse enzymatique de l'acétylthiocholine, au moyen d'un lecteur de microplaques (TECAN Infinite M200, Lyon, France) à la longueur d'onde de 412 nm chaque minute durant 10 minutes. Le donépézil est utilisé comme produit de référence.

Pour les composés présentant une inhibition de l'acétylcholinestérase significative (≥ 50%) après 4 min de réaction, les valeurs d'IC₅₀ sont mesurées graphiquement à partir de 6 points de la courbe d'inhibition au moyen du logiciel Origin.

### Résultats

A titre d'exemples les activités inhibitrices de l'acétylcholinestérase humaine de plusieurs composés selon l'invention vis-à-vis des récepteurs 5-HT4 sont reportées dans le tableau 6.

**Tableau 6 : inhibition de l'acétylcholinestérase humaine de plusieurs composés selon l'invention, le donépézil étant pris comme référence (IC₅₀ en nM).**

| composé | IC₅₀ en nM |
|---|---|
| **1*** | 748 |
| **2*** | 445 |
| **3*** | 658 |
| **4** | 26 |
| **5** | 24 |
| **6** | 937 |
| **7** | 577 |
| **8** | 69 |
| **9** | 63 |
| **10** | 201 |
| **11** | 321 |
| **12** | 57 |
| **13** | 118 |
| **14** | 99 |
| **15** | 304 |
| **16** | 222 |
| **17 *** | 3730 |
| **18 *** | 8700 |
| **19 *** | 5090 |
| **20 *** | 2720 |
| **21** | 411 |
| **22** | 395 |
| **23** | 625 |
| **24** | 320 |
| **donépézil** | 11 |

| | |
|---|---|
| * : Non-couvert par l'invention | |

### D) test de déplacement du propidium

### Matériel et méthodes

Le diiodure de propidium lorsqu'il se lie au site périphérique de l'AChE produit une augmentation de fluorescence qui peut être utilisée comme preuve de sa liaison à l'enzyme. Les mesures de fluorescence sont effectuées dans 200 µL de solution en plaques 96 puits grâce à un lecteur de microplaques Tecan Infinite M200. Cinq unités d'eeAChE sont incubées pendant 15 min à 25°C dans du tampon 1 mM Tris/HCl, pH 8,0 avec 150 µL de solution 10⁻⁵M de composés à tester ou de donépézil comme référence. 50 µL de solution micromolaire de diiodure de propidium sont ajoutés 10 min avant la mesure de fluorescence. La longueur d'onde d'excitation est mesurée à 535 nm, celle d'émission à 595 nm. Chaque essai est répété au moins trois fois.

### Résultats

A titre d'exemples les valeurs de déplacement du diiodure de propidium du site périphérique de l'eeAChE de plusieurs composés selon l'invention sont reportées dans le tableau 7.

**Tableau 7 : déplacement du diiodure de propidium du site périphérique de l'eeAChE de plusieurs composés selon l'invention (%)**

| composé | % déplacement propidium |
|---|---|
| **5** | 22% |
| **8** | 20% |
| **9** | 22% |
| **12** | 20% |
| **14** | 20% |
| **16** | 21% |
| **23** | 22% |
| **donépézil** | 23% |

### E) test de reconnaissance d'objet

### Matériel et méthodes

### Animaux

Les tests sont effectués avec des souris mâles de souche NMRI âgées d'environ 3 mois au moment des tests. Ces souris sont hébergées par groupes de 15 dans des cages standard en polycarbonate (42 x 29 x 15 cm³), dans une animalerie dont la température est maintenue à 22 ± 2°C et le pourcentage d'humidité à 55 ± 10%. Elles ont un libre accès à la nourriture et à l'eau et sont maintenues en cycle inversé (phase de lumière entre 20h00 et 8h00) afin de pouvoir réaliser les expériences durant leur phase d'activité.

### Test de reconnaissance d'objet

Le test de reconnaissance d'objet est basé sur la préférence spontanée des rongeurs pour la nouveauté, sans mettre en jeu de privation de nourriture ni de choc électrique. C'est un test qui permet d'évaluer une forme de mémoire épisodique chez le rongeur dans le sens où l'on évalue la capacité de l'animal à mémoriser une information liée à un contexte spatio-temporel (Dere et al., 2007).

Le dispositif se compose d'une enceinte en PVC carrée (33 x 33 x 20 cm, illuminée à 10 lux au centre) dont les surfaces sont noires. Le test se déroule en 2 phases : l'une de familiarisation, permettant aux animaux de se familiariser avec l'environnement du test (enceinte, pièce, expérimentateur), l'autre d'évaluation des performances de mémoire. Une caméra est située au dessus de l'appareillage, elle est reliée à un système de video-tracking (ViewPoint®). Lors des tests comportementaux, l'expérimentateur ne se trouve pas dans la pièce d'expérimentation mais dans une pièce adjacente où se trouve le système de video-tracking.

Après une phase de familiarisation (jour 1, exposition individuelle pendant 5 minutes à l'open-field sans objet) la phase d'évaluation des performances de mémoire débute le 2ème jour et est elle-même composée de 2 sessions, l'une de présentation, l'autre de test. Immédiatement avant la session de présentation, la souris est familiarisée à nouveau à l'appareillage pendant une minute, puis deux objets identiques, notés A1 et A2, sont placés à environ 5 cm des murs de l'enceinte. La souris est placée dos aux objets, face à l'expérimentateur, et explore librement le dispositif. La session est arrêtée lorsque la souris atteint un temps total d'exploration des deux objets de 30 secondes. Le temps d'exploration de chaque objet, respectivement a1 et a2, ainsi que le temps pour atteindre le critère d'exploration sont mesurés. Après un délai inter-sessions de 24 heures, une session de test est réalisée. Deux objets sont utilisés : un objet A3 familier (une copie de A1 ou A2 non présenté précédemment) et un objet B, différent de l'objet A, qui correspondra à l'objet nouveau. La session est arrêtée par l'expérimentateur lorsque la souris atteint un critère d'exploration totale de 30 secondes. Le temps pour atteindre le critère d'exploration ainsi que le temps d'exploration de l'objet nouveau sont mesurés et ce dernier est comparé au niveau chance (15 s).

Les objets utilisés lors du jour test sont un dauphin bleu en céramique (4 cm de diamètre et 10 cm de haut) et un flacon transparent rempli de sable (3 x 4,5 x 9 cm), disponibles en quatre exemplaires, qui sont nettoyés après chaque utilisation (Ethanol 70%). Ces objets sont maintenus avec de la Patafix® au niveau du plancher de l'open field de manière à ce que les souris ne puissent pas les déplacer. Les combinaisons d'objets (flacon/flacon et dauphin/dauphin) et l'emplacement du nouvel objet (gauche/droite) sont déterminés aléatoirement pour éviter les biais dus à une éventuelle préférence d'objet ou de place.

Les résultats sont exprimés par le pourcentage d'exploration de chaque objet pendant la session test. Les données sont analysées par une analyse de variance (ANOVA) avec mesures répétées, suivie d'un test de comparaison multiple de Student-Newman-Keuls (SNK) .

### Administration des composés

Des lots de 10 à 12 souris sont constitués par groupe expérimental. Les composés en solution dans le sérum physiologique sont administrés par voie intrapéritonéale (volume d'injection, 10 ml/kg de poids corporel), 30 minutes avant la session d'acquisition. Un lot contrôle (sérum physiologique) est constitué.

### Résultats (figure 1)

A titre d'exemple, le composé **9** induit une amélioration des performances de reconnaissance d'objets par rapport au lot contrôle avec un effet maximal dès la dose de 0,3 mg/kg (figure 1).

## Revendications

1. Composé de formule générale (I) : 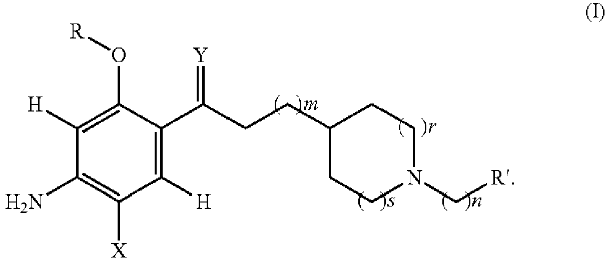 dans laquelle :
X représente
un atome d'hydrogène, ou
un atome d'halogène (Hal), (Hal) désignant le fluor, le chlore, le brome ou l'iode, ou
un groupement polyhalogénoalkyl Cₚ(Hal)₂ₚ₊₁, en chaîne droite ou ramifiée, avec p = 1, 2, 3 ou 4, (Hal) ayant la même signification que ci-dessus ;
Y représente
un atome d'oxygène, ou
un atome de soufre, ou
un radical N-R" où R" représente un atome d'hydrogène, un radical -OH, un radical alkyl C_{q}H_{2q+1}, en chaîne droite ou ramifiée, avec q = 1, 2, 3 ou 4 ;
( )m représente un nombre m de groupes méthylène, dont la valeur est 1, 2 ou 3 ;
( )n représente un nombre n de groupes méthylène, dont la valeur est 0, 1, 2 ou 3 ;
( )r et ( )s représentent un nombre r et s respectivement de groupe(s) méthylène, dont les valeurs sont : r = s = 0 ou r = s = 1 ou r = s = 2 ou r = 0 et s = 1 ou enfin r = 0 et s = 2 ;
R représente
un atome d'hydrogène, ou
un groupe alkyl en C1-C5 en chaîne droite ou ramifiée pouvant porter un ou plusieurs atomes de F ;
R' représente
un groupe cycloalkyl en C3-C10 pouvant porter un ou plusieurs groupements R et pouvant posséder un atome d'oxygène, ou un atome d'azote pouvant être substitué par R,
ainsi que ses énantiomères ou diastéréoisomères et ses racémiques, ses sels d'acide, ses hydrates ou ses produits de solvatation.

2. Composé selon la revendication 1, **caractérisé en ce que**, dans sa formule (I), X représente un atome d'halogène.

3. Composé selon la revendication 1, **caractérisé en ce que**, dans sa formule (I), Y représente un atome d'oxygène.

4. Composé selon la revendication 1, **caractérisé en ce que**, dans sa formule (I), tous les coefficients m, n, r et s ont la valeur 1.

5. Composé selon la revendication 1, **caractérisé en ce que**, dans sa formule (I), R représente H, CH₃, CH₂CH₃ ou CH₂-CH₂F.

6. Composé selon la revendication 1, **caractérisé en ce que**, dans sa formule (I), R' représente un radical pris dans le groupe formé par les radicaux cyclopropyl, cyclobutyl, cyclopentyl, cyclohexyl, cycloheptyl et 4-pipéridine.

7. Composé selon les revendications 2 à 6, **caractérisé en ce que**, dans sa formule (I), R représente un radical méthyl et R' un radical cycloalkyl en C₄-C₇.

8. Composé **caractérisé en ce que** ledit composé est le 2-chloro-4-[[2-[1-(cyclohexylméthyl)-4-pipéridyl]éthyl]-N-méthoxycarbonimidoyl]-5-méthoxyaniline.

9. Procédé de préparation des composés selon la revendication 1, dans la formule (I) desquels R = H et X, Y, m, n, r, s et R' ont les significations indiquées dans la revendication 1, **caractérisé en ce que** l'on fait réagir un composé selon la revendication 1, dans la formule (I) duquel R représente un groupe méthyl avec du chlorure d'aluminium en présence d'iodure de sodium dans l'acétonitrile, pour obtenir le composé de formule (I) désiré.

10. Procédé de préparation des composés selon la revendication 1, dans la formule (I) desquels, R représente un radical alkyl en C1-C3, en chaine droite ou ramifiée, pouvant porter un ou plusieurs atomes de fluor, X, Y, m, n, r, s, et R' ayant les significations indiquées dans la revendication 1, **caractérisé en ce que** l'on fait réagir un composé selon la revendication 1, dans lequel R = H, X, Y, m, n, r, s, et R' ayant les significations indiquées dans la revendication 1, avec un halogénure ou un tosylate d'alkyl en C1-C3, en chaine droite ou ramifiée, pouvant porter un ou plusieurs atomes de fluor, en présence de carbonate de potassium, dans un solvant approprié.

11. Procédé de préparation de composés de formule (I) telle que décrite dans la revendication 1, dans lesquels Y est un groupe oxime éventuellement O-substitué, X, m, n et R' étant tels qu'indiqués dans la revendication 1, **caractérisé en ce que** l'on fait réagir un composé selon la revendication 1, dans la formule (I) duquel X, m, n et R' ont les significations indiquées ci-dessus et Y = O, avec un sel d'hydroxylamine O-substituée en présence de carbonate de calcium dans un mélange éthanol/eau, pour obtenir le composé de formule (I) désiré.

12. Composition pharmaceutique **caractérisée en ce qu'**elle contient, à titre de principe actif, au moins un composé selon l'une des revendications 1 à 8.

13. Composition pharmaceutique selon la revendication 12, **caractérisée en ce qu'**elle contient un excipient pharmaceutiquement acceptable.

14. Composition pharmaceutique selon l'une des revendications 12 ou 13 **caractérisée en ce que** le(s) composé(s) de formule (I) qu'elle contient, est (sont) un (des) sel(s) pharmaceutiquement acceptable(s).

15. Composition pharmaceutique selon l'une des revendications 12 à 14, **caractérisée en ce qu'**elle contient au moins un principe actif ayant une action inhibitrice d'acétylcholinestérase et choisi dans le groupe formé par :
a) la 1,2,3,4-Tétrahydroacridin-9-amine
b) le (RS)-2-[(1-benzyl-4-pipéridyl)méthyl-5,6-diméthoxy-2,3-dihydroindén-1-one
c) la (S)-N-éthyl-N-méthyl-3-[(1-diméthylamino)éthyl]-phényl carbamate et
d) la 4aS,6R,8aS)-5,6,9,10,11,12-hexahydro-3-méthoxy-11-méthyl-4aH-[1]benzofuro[3a,3,2-ef][2]benzazépin-6-ol

16. Composition pharmaceutique selon l'une des revendications 12 à 15, **caractérisée en ce qu'**elle contient, au moins un agoniste partiel des récepteurs 5-HT4 choisi dans le groupe formé par :
a) le 1-(4-amino-5-chloro-2-méthoxyphényl)-2-[1-butyl-4-piperidyl]propan-1 one,
b) le N-(2-(4-(3-(4-amino-5-chloro-3-méthoxyphényl)-3-oxypropyl)pipéridin-1-yl)éthyl)méthane sulfonamide,
ledit agoniste étant dans une proportion comprise entre 10/90 et 90/10 par rapport au(x) principe(s) actif(s) constitué(s) par un composé selon l'une des revendications 1 à 8.

17. Composition pharmaceutique selon l'une des revendications 12 à 16, pour son utilisation dans le traitement des maladies neurologiques avec déficit amnésique chez des mammifères.

18. Composition pharmaceutique selon la revendication 17 pour son utilisation dans le traitement de la maladie d'Alzheimer chez un sujet humain.

19. Composition pharmaceutique selon l'une des revendications 12 à 16, dans laquelle le principe actif est conditionné sous forme de mélange avec des agents de dispersion, des agents mouillants, des agents de mise en suspension, des édulcorants ou des correcteurs de goût.

## Patentansprüche

1. Verbindung der allgemeinen Formel (I): 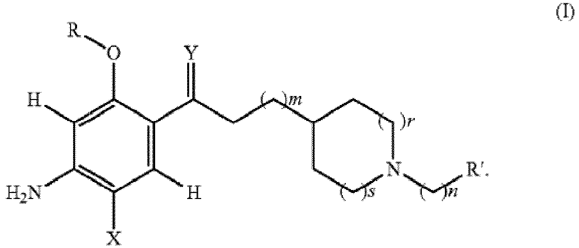 in der:
X Folgendes repräsentiert
ein Wasserstoffatom, oder
ein Halogenatom (Hal), wobei (Hal) Fluor, Chlor, Brom oder Jod bezeichnet, oder
eine Polyhalogenalkyl-Gruppierung Cₚ(Hal)₂ₚ₊₁, in gerader oder verzweigter Kette, mit p = 1, 2, 3 oder 4, wobei (Hal) dieselbe Bedeutung wie oben aufweist;
Y Folgendes repräsentiert
ein Sauerstoffatom, oder
ein Schwefelatom, oder
ein N-R" Radikal, wobei R" ein Wasserstoffatom, ein -OH Radikal, ein Alkyl C_{q}H_{2q+1} Radikal, in gerader oder verzweigter Kette, mit q = 1, 2, 3 oder 4 repräsentiert;
()m eine Anzahl m an Methylen-Gruppen repräsentiert, deren Wert 1, 2 oder 3 ist;
()n eine Anzahl n an Methylen-Gruppen repräsentiert, deren Wert 0, 1, 2 oder 3 ist;
()r und ()s eine Anzahl r und s jeweils von Methylen Gruppe(n) repräsentieren, deren Werte sind: r = s = 0 oder r = s = 1 oder r = s = 2 oder r = 0 und s = 1 oder schließlich r = 0 und s = 2;
R Folgendes repräsentiert
ein Wasserstoffatom, oder
eine Alkyl-Gruppe mit C1-C5, in gerader oder verzweigter Kette, die ein oder mehrere F-Atome tragen kann;
R' Folgendes repräsentiert
eine Cycloalkyl-Gruppe mit C3-C10, die eine oder mehrere R-Gruppierungen tragen kann und ein Sauerstoffatom oder ein Stickstoffatom besitzen kann das durch R substiuiert wird,
sowie deren Enantiomere oder Diastereoisomere und deren Racemate, deren Säuresalze, deren Hydrate oder deren Solvatisierungsprodukte.

2. Verbindung nach Anspruch 1, **dadurch gekennzeichnet, dass** in ihrer Formel (I) X ein Halogenatom repräsentiert.

3. Verbindung nach Anspruch 1, **dadurch gekennzeichnet, dass** in ihrer Formel (I) Y ein Sauerstoffatom repräsentiert.

4. Verbindung nach Anspruch 1, **dadurch gekennzeichnet, dass** in ihrer Formel (I) alle Koeffizienten m, n, r und s den Wert 1 aufweisen.

5. Verbindung nach Anspruch 1, **dadurch gekennzeichnet, dass** in ihrer Formel (I) R H, CH₃, CH₂CH₃ oder CH₂-CH₂F repräsentiert.

6. Verbindung nach Anspruch 1, **dadurch gekennzeichnet, dass** in ihrer Formel (I) R' ein Radikal repräsentiert, das aus der Gruppe entnommen ist, die durch die Cyclopropyl-, Cyclobutyl-, Cyclopentyl-, Cyclohexyl-, Cycloheptyl- und 4-Piperidin-Radikale gebildet wird.

7. Verbindung nach den Ansprüchen 2 bis 6, **dadurch gekennzeichnet, dass** in ihrer Formel (I) R ein Methyl-Radikal repräsentiert, und R' ein Cycloalkyl mit C₄-C₇.

8. Verbindung, **dadurch gekennzeichnet, dass** die Verbindung 2-Chlor-4-[[2-[1-(cyclohexymethyl)-4-piperidyl]ethyl]-N-methoxycarbonimidoyl]-5-methoxyanlilin ist.

9. Verfahren zur Herstellung der Verbindungen nach Anspruch 1, in deren Formel (I) R = H ist und X, Y, m, n, r, s und R' Bedeutungen aufweisen, die im Anspruch 1 angegeben sind, **dadurch gekennzeichnet, dass** man eine Verbindung nach Anspruch 1, in deren Formel (I) R eine Methylgruppe mit Aluminiumchlorid repräsentiert im Beisein von Natriumiodid in Acetonitril reagieren lässt, um die gewünschte Verbindung der Formel (I) zu erhalten.

10. Verfahren zur Herstellung der Verbindungen nach Anspruch 1, in deren Formel (I) R ein Alkyl-Radikal mit C1-C3, in gerader oder verzweigter Kette, repräsentiert, das ein oder mehrere Fluoratome tragen kann, wobei X, Y, m, n, r, s und R' Bedeutungen aufweisen, die im Anspruch 1 angegeben sind, **dadurch gekennzeichnet, dass** man eine Verbindung nach Anspruch 1, in der R = H, X, Y, m, n, r, s und R' die Bedeutungen aufweisen, die im Anspruch 1 angegeben sind, mit einem Halogenid oder einem Alkyltosylat mit C1-C3, in gerader oder verzweigter Kette, das ein oder mehrere Fluoratome tragen kann, im Beisein von Kalium in einem geeigneten Lösungsmittel reagieren lässt.

11. Verfahren zur Herstellung von Verbindungen der Formel (I) nach Anspruch 1, in denen Y eine eventuell O-substiruierte Oxim-Gruppe ist, X, m, n und R' wie in Anspruch 1 angegeben sind, **dadurch gekennzeichnet, dass** man eine Verbidung nach Anspruch 1, in deren Formel (I) X, m, n und R' die Bedeutungen wie oben aufweisen und Y = O ist, mit einem O-substituierten Hydroxylaminsalz im Beisein von Calciumcarbonat in einem Ethanol-/WasserGemisch reagieren lässt, um die gewünschte Verbindung der Formel (I) zu erhalten.

12. Pharmazeutische Zusammensetzung, **dadurch gekennzeichnet, dass** sie als Wirkstoff mindestens eine Verbindung nach einem der Ansprüche 1 bis 8 enthält.

13. Pharmazeutische Zusammensetzung nach Anspruch 12, **dadurch gekennzeichnet, dass** sie einen pharmazeutisch annehmbaren Hilfsstoff enthält.

14. Pharmazeutische Zusammensetzung nach einem der Ansprüche 12 oder 13, **dadurch gekennzeichnet, dass** die Verbindung(en) der Formel (I), die sie enthält pharmazeutisch annehmbare(s) Salz(e) ist (sind).

15. Pharmazeutische Zusammensetzung nach einem der Ansprüche 12 bis 14, **dadurch gekennzeichnet, dass** sie mindestens einen Wirkstoff enthält, der eine Acetylcholinesterase unterdrückende Wirkung aufweist und aus der Gruppe ausgewählt ist, die gebildet ist durch:
a) 1,2,3,4-Tetrahydroacridin-9-Amin
b) (RS)-2-[(1-Benzyl-4-Piperidyl)methyl-5,6-Dimethoxy-2,3-Dihydroinden-1-one
c) (S)-N-Ethyl-N-Methyl-3-[(1-Dimethylamino)ethyl]-phenylcarbamat und
d) 4aS,6R,8aS)-5,6,9,10,11,12-hexahydro-3-methoxy-11-methyl-4aH-[1]benzofuro[3a,3,2-ef][2]benzazepin-6-ol

16. Pharmazeutische Zusammensetzung nach einem der Ansprüche 12 bis 15, **dadurch gekennzeichnet, dass** sie mindestens einen Partialagonisten der 5-HT4-Rezeptoren enthält, ausgewählt aus der Gruppe, die gebildet ist durch:
a) 1-(4-Amino-5-Chlor-2-Methoxyhenyl)-2-[1-butyl-4-piperidyl]propan-1-one
b) N-(2-(4-(3-(4-amino-5-chlor-3-methoxyphenyl)-3-oxypropyl)piperidin-1-yl)ethyl)methansulfonamid,
wobei der Agonist in einer Proportion ist, die zwischen 10/90 und 90/10 im Verhältnis zu dem (den) Wirkstoff(en), der (die) durch eine Verbindung nach einem der Ansprüche 1 bis 8 gebildet wird (werden).

17. Pharmazeutische Zusammensetzung nach einem der Ansprüche 12 bis 16 für deren Verwendung bei der Behandlung neurologischer Erkrankungen mit mnestischem Defizit bei den Säugern.

18. Pharmazeutische Zusammensetzung nach Anspruch 17, für deren Verwendung bei der Behandlung der Alzheimer-Erkrankung bei einem menschlichen Patienten.

19. Pharmazeutische Zusammensetzung nach einem der Ansprüche 12 bis 16, deren Wirkstoff in Form eines Gemisches mit Dispergiermitteln, Netzmitteln, Suspendierungsmitteln, Süßstoffen oder Geschmackskorrektoren konditioniert wird.

## Claims

1. Compound of the general formula (I): 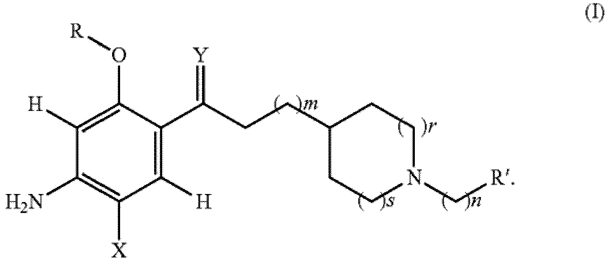 wherein:
X represents
a hydrogen atom, or
a halogen atom (Hal), where (Hal) is fluorine, chlorine, bromine or iodine, or
a straight- or branched-chain Cₚ(Hal)₂ₚ₊₁ polyhalogenoalkyl group, where p = 1, 2, 3 or 4, (Hal) having the same meaning as indicated above;
Y represents
an oxygen atom, or
a sulfur atom, or
an N-R" radical where R" represents a hydrogen atom, an -OH radical, a straight- or branched-chain C_{q}H_{2q+1} alkyl radical, where q = 1, 2, 3 or 4 ;
()m represents a number m of methylene group(s), whose value is 1, 2 or 3
()n represents a number n of methylene group(s), whose value is 0, 1, 2 or 3
()r and ()s represent a number r and s respectively of methylene group(s), whose values are: r = s = 0; orr = s = 1; orr = s = 2; orr = 0 and s = 1; or lastly r = 0 and s = 2;
R represents
a hydrogen atom, or
a straight- or branched-chain C1-C5 alkyl group, capable of carrying one or more F atoms
R' represents
a C3-C10 cycloalkyl capable of carrying one or more R groups and of possessing an oxygen atom, or a nitrogen atom that can be substituted by R,
as well as its enantiomers or diastereoisomers and its racemics, its acid salts, its hydrates or its solvation products.

2. Compound according to claim 1, **characterized in that**, in its formula (I), X represents a halogen atom.

3. Compound according to claim 1, **characterized in that**, in its formula (I), Y represents an oxygen atom.

4. Compound according to claim 1, **characterized in that**, in its formula (I), all of the coefficients m, n, r and s have the value 1.

5. Compound according to claim 1, **characterized in that**, in its formula (I), R represents H, CH₃, CH₂CH₃ or CH₂-CH₂F.

6. Compound according to claim 1, **characterized in that**, in its formula (I), R' represents a radical taken from the group formed by the radicals cyclopropyl, cyclobutyl, cyclopentyl, cyclohexyl, cycloheptyl and 4-piperidine.

7. Compound according to claims 2 to 6, **characterized in that**, in its formula (I), R represents a methyl radical and R' a C₄-C₇ cycloalkyl radical.

8. Compound **characterized in that** said compound is 2-chloro-4-[[2-[1-(cyclohexylmethyl)-4-piperidyl]ethyl]-N-methoxycarbonimidoyl]-5-methoxyaniline.

9. Method of preparing the compounds according to claim 1, in formula (I), wherein R = H and X, Y, m, n, r, s and R' have the meanings indicated in claim 1, **characterized in that** a compound according to claim 1, in formula (I), wherein R represents a methyl group is reacted with aluminum chloride in the presence of sodium iodide in acetonitrile to obtain the desired compound of formula (I).

10. Method of preparing the compounds according to claim 1, in formula (I), wherein R represents a straight- or branched-chain C1-C3 alkyl radical, capable of carrying one or more fluorine atoms, X, Y, Z, m, n, r, s, and R' having the meanings indicated in claim 1, **characterized in that** a compound according to claim 1, wherein R = H and X, Y, m, n, r, s, and R' having the meanings indicated in claim 1, is reacted with a halide or a straight- or branched-chain C1-C3 alkyl tosylate, capable of carrying one or more fluorine atoms, in the presence of potassium carbonate, in an appropriate solvent.

11. Method of preparing the compounds of formula (I) as described in claim 1, wherein Y is a possible O-substituted oxime group, X, m, n and R' being as indicated in claim 1, **characterized in that** a compound according to claim 1, in formula (I), of which X, m, n and R' have the meanings indicated above and Y = O, is reacted with an O-substituted hydroxylamine salt in the presence of calcium carbonate in an ethanol/water mixture, to obtain the desired compound of formula (I).

12. Pharmaceutical composition **characterized in that** it contains, as an active ingredient, at least one compound according to one of claims 1 to 8.

13. Pharmaceutical composition according to claim 12, **characterized in that** it contains a pharmaceutically acceptable excipient.

14. Pharmaceutical composition according to one of claims 12 or 13, **characterized in that** the compound(s) of formula (I) that the composition contains, is (are) pharmaceutically acceptable.

15. Pharmaceutical composition according to one of claims 12 to 14, **characterized in that** it contains at least one active ingredient having an inhibitory action of acetylcholinesterase and chosen from the group formed by:
a) 1,2,3,4-Tetrahydroacridin-9-amine
b) (RS)-2-[(1-benzyl-4-piperidyl)methyl-5,6-dimethoxy-2,3-dihydroinden-1-one
c) (S)-N-ethyl-N-methyl-3-[(1-dimethylamino)ethyl]-phenyl carbamate and
d) 4aS,6R,8aS)-5,6,9,10,11,12-hexahydro-3-methoxy-11-methyl-4aH-[1]benzofuro[3a,3,2-ef][2]benzazepin-6-ol

16. Pharmaceutical composition according to one of claims 12 to 15, **characterized in that** it contains at least one partial 5-HT4 receptor agonist chosen from the group formed by:
a) 1-(4-amino-5-chloro-2-methoxyphenyl)-2-[1-butyl-4-piperidyl]propan-1 one,
b) N-(2-(4-(3-(4-amino-5-chloro-3-methoxyphenyl)-3-oxypropyl)piperidin-1-yl)ethyl)methane sulfonamide,
the said agonist being in a proportion of between 10/90 and 90/10 in relation to the active ingredient(s) formed by at least one compound according to one of claims 1 to 8.

17. Pharmaceutical composition according to one of claims 12 to 16, for use in the treatment of neurological diseases with amnestic deficit in mammals.

18. Pharmaceutical composition according to claim 17, for use in the treatment of Alzheimer's disease in a human subject.

19. Pharmaceutical composition according to one of claims 12 to 16, wherein the active ingredient is packaged in the form of a mixture with dispersion agents, softening agents, suspension agents, sweeteners or flavor enhancers.
